(19) Europäisches Patentamt / European Patent Office / Office européen des brevets

(11) **EP 4 002 376 A1**

(12) **EUROPEAN PATENT APPLICATION**

(43) Date of publication:
**25.05.2022 Bulletin 2022/21**

(21) Application number: **21196277.4**

(22) Date of filing: **13.09.2021**

(51) International Patent Classification (IPC):
**G16C 20/30** (2019.01)  **G16C 60/00** (2019.01)
**G16C 20/70** (2019.01)

(52) Cooperative Patent Classification (CPC):
**G06N 5/003; G06N 7/005; G06N 20/20;**
**G16C 20/30; G16C 60/00;** G16C 20/70

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.11.2020 JP 2020193402**

(71) Applicant: **FUJITSU LIMITED**
**Kanagawa 211-8588 (JP)**

(72) Inventor: **Shioga, Takeshi**
**Kanagawa, 211-8588 (JP)**

(74) Representative: **Hoffmann Eitle**
**Patent- und Rechtsanwälte PartmbB**
**Arabellastraße 30**
**81925 München (DE)**

(54) **MIXTURE PHYSICAL PROPERTY IDENTIFICATION METHOD, MIXTURE PHYSICAL PROPERTY IDENTIFICATION APPARATUS, AND MIXTURE PHYSICAL PROPERTY IDENTIFICATION PROGRAM**

(57)    A mixture physical property identification method for a computer to execute a process includes, creating a prediction term for predicting at least one physical property of a mixture of a plurality of candidate substances; and identifying the physical property of the mixture, when the first learning datasets and the corresponding datasets do not demonstrate the certain correlation, obtaining virtual datasets based on an integration model, and setting at least some of the virtual datasets as second learning datasets, and comparing the first learning datasets with corresponding datasets corresponding to the first learning datasets in a second prediction model based on the second learning datasets, when the first learning datasets and the corresponding datasets demonstrate the certain correlation, the prediction term is created based on regression coefficients of the respective candidate substances obtained from the second prediction model.

FIG. 1

EP 4 002 376 A1

**Description**

FIELD

The embodiments discussed herein are related to a mixture physical property identification method, a mixture physical property identification apparatus, and a mixture physical property identification program.

BACKGROUND

In the related art, for an insulating refrigerant having no electrical conductivity, a mixture of a plurality of candidate substances has been used, and physical properties (physical properties and attributes) of the mixture have been tried to be optimized by optimizing a combination of the kinds of the candidate substances and a component ratio of the candidate substances.

Efficient optimization of the physical properties of a mixture of a plurality of candidate substances requests accurate prediction of the physical properties of the mixture. An example of a method of predicting a physical property of a mixture is a method of calculating a physical property of a mixture based on a combination of kinds of candidate substances and a component ratio of the candidate substances.

As the related art for using this method, for example, there has been proposed a method using a mathematical expression capable of estimating a physical property in a mixed state (physical property estimating equation) based on the physical property of the candidate substances. In the related art, an objective function expression for predicting the physical property of a mixture is defined by using such a mathematical expression capable of estimating a physical property in a mixed state, and the physical property of the mixture is predicted and optimized by optimizing the objective function expression.

However, in this related art, in a case of predicting a physical property (performance) for which a mathematical expression capable of estimating a physical property in a mixed state does not exist, there is a problem that an objective function expression for optimizing the physical property of a mixture is so difficult to construct that the physical property of the mixture may not be identified.

For a mixture such as a vulcanized rubber composition or a melt obtained by casting, there has been proposed a technique in which, for optimizing a physical property of the mixture, the physical property of the mixture is predicted by using machine learning and a combination (component contents) of materials in the mixture is determined.

However, in this related art, there are problems that the prediction accuracy of the physical property of the mixture is sometimes insufficient and that it is difficult to improve the prediction accuracy of the physical property of the mixture.

As described above, in the related art, there are problems that it is difficult to predict a physical property (performance) for which a mathematical expression capable of estimating a physical property in a mixed state (physical property estimating equation) does not exist, and that it is difficult to improve prediction accuracy of a physical property of a mixture in a case of using machine learning.

Japanese Laid-open Patent Publication Nos. 2020-030680 and 2019-195838 are disclosed as related art.

Shuzo Ohe, Physical Property Estimation Method (Japanese), Data Book Shuppan-sha is also disclosed as related art.

SUMMARY

[TECHNICAL PROBLEM]

However, with the above techniques, it is difficult to specify the cause of the accuracy deterioration in the model. For example, the technique is not able to specify the case of the mistake for the presentation of wrong data, and is not able to specify the cause related to the accuracy deterioration in the presentation of the domain shift.

In one aspect, it is aimed to provide a data generation program, a data generation method, and an information processing device capable of specifying the cause of accuracy deterioration in a model.

[SOLUTION TO PROBLEM]

In one aspect, an object of the present disclosure is to provide a mixture physical property identification method and the like capable of predicting and identifying a physical property of a mixture with high accuracy even in a case of predicting the physical property for which a mathematical expression capable of estimating a physical property in a mixed state (physical property estimating equation) does not exist.

In one aspect, the present disclosure may provide a mixture physical property identification method and the like capable of predicting and identifying a physical property of a mixture with high accuracy even in a case of predicting the physical property for which a mathematical expression capable of estimating a physical property in a mixed state (physical property estimating equation) does not exist.

[ADVANTAGEOUS EFFECTS OF INVENTION]

According to one embodiment, the cause of accuracy deterioration in a model may be specified.

BRIEF DESCRIPTION OF DRAWINGS

FIG. 1 is a diagram illustrating an example of how to select a combination of candidate substances when a plurality of candidate substances are mixed to produce a mixture;
FIG. 2 illustrates an example of a flowchart of optimizing a physical property (performance) of a mixture by using a technique using a physical property estimating equation;
FIG. 3A is a diagram illustrating an example of composition-by-composition prediction models of a plurality of kinds of mixtures based on distributions of datasets on a physical property (physical property value datasets);
FIG. 3B is a diagram illustrating an example of a relationship among the physical property value of a mixture of A, B, and C, a percentage of A, and a percentage of B in FIG. 3A;
FIG. 3C is a diagram illustrating an example of a relationship among the physical property value of a mixture of C, D, and E, a percentage of C, and a percentage of D in FIG. 3A;
FIG. 3D is a diagram illustrating an example of a Gaussian mixture model in which the composition-by-composition prediction models of the plurality of kinds of mixtures illustrated in FIG. 3A are integrated and combined together;
FIG. 4 is a diagram illustrating a hardware configuration example of a mixture physical property identification apparatus disclosed herein;
FIG. 5 is a diagram illustrating another hardware configuration example of the mixture physical property identification apparatus disclosed herein;
FIG. 6 is a diagram illustrating a functional configuration example of the mixture physical property identification apparatus disclosed herein;
FIG. 7A and FIG. 7B illustrate an example of a flowchart of identifying and optimizing a physical property of a mixture by using an example of the technique disclosed herein;
FIG. 8 is a diagram illustrating an example of a functional configuration of an annealing machine for use in an annealing method;
FIG. 9 is a diagram illustrating an example of an operation flow of a transition control unit;
FIG. 10 is a diagram illustrating an example of a distribution of thermal conductivity of 40 mixtures obtained by a non-equilibrium molecular dynamics simulation;
FIG. 11 is a diagram illustrating an example of a relationship between prediction values calculated from a prediction model constructed by using 32 learning datasets and actual values (learning datasets);
FIG. 12 is a diagram illustrating an example of a relationship between the number of virtual datasets generated and RMSE/MAE in a thermal conductivity prediction model (second prediction model) constructed by using 80% of the generated virtual datasets as learning datasets; and
FIG. 13 is a diagram illustrating an example of a relationship between prediction values calculated by using a prediction model constructed by using 1600 virtual datasets as learning datasets among 2000 virtual datasets and actual values (learning datasets) corresponding to the prediction values.

DESCRIPTION OF EMBODIMENTS

(Mixture Physical Property Identification Apparatus)

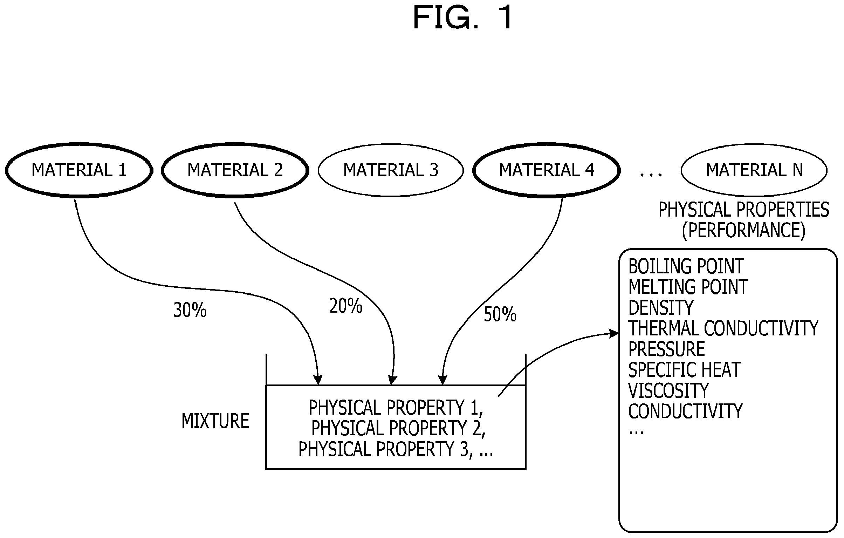

The technique disclosed herein is based on the inventor's finding that, in the related art, it is difficult to predict a physical property (performance) for which a mathematical expression capable of estimating a physical property in a mixed state (physical property estimating equation) does not exist and it is difficult to improve the prediction accuracy of a physical property of a mixture in a case of using machine learning. Therefore, problems and others of the related art will be described in more detail before describing the details of the technique disclosed herein.
First, physical properties of a mixture such as a mixed refrigerant may be determined, for example, based on a combination of kinds of candidate substances forming the mixture and a component ratio of the candidate substances.
Here, for example, considered is a case where, as illustrated in FIG. 1, a predetermined number of materials are selected and mixed from N kinds of materials including a material 1, a material 2, a material 3, a material 4, ..., and a material N, which are candidate substances, and a plurality of physical properties (performance depending on intended use) of the mixture are optimized. In the example illustrated in FIG. 1, in selection of three materials from among the N kinds of materials, a search for a combination of kinds of materials and a component ratio (mixture ratio) thereof is performed so that desired physical properties of the mixture become high. As illustrated in FIG. 1, examples of the physical properties (performance) of the mixture include boiling point, melting point, density, thermal conductivity, pressure, specific heat, viscosity, an electrical conductivity, and so on, and some physical properties desired to be optimized in the mixture are selected from these physical properties and then optimized.

For execution of such an optimization, it is possible to use, for example, an objective function (cost function or energy function) in which physical properties of a mixture are defined as parameters and optimize the physical properties (performance) of the mixture by optimizing (minimizing or maximizing) the objective function. An objective function expression representing an objective function for optimizing physical properties of a mixture in the form of an expression is, for example, as follows:

$$E = \alpha \cdot [\text{Physical Property 1}] + \beta \cdot [\text{Physical Property 2}] + \gamma \cdot [\text{Physical Property 3}] + ... + \text{Constraint Term},$$

where E is an objective function expression and $\alpha$, $\beta$, and $\gamma$ are weighting coefficients for the respective physical properties. The constraint term is a term that represents a constraint such as the number of selected materials (substances) in the objective function expression.

In the above objective function expression, [Physical Property 1] to [Physical Property N] are physical property values as design targets of a mixture, which represent specific physical properties (individual specifications of performance) desired to be optimized in order to maximize the physical properties depending on intended use of the mixture, and may be physical property values such as thermal conductivity and specific heat, for example. A weighting coefficient is assigned to each physical property value in the above objective function expression, and it is possible to set which of the physical property values more importance (heavier weight) is given to by changing the weights (coefficients $\alpha$, $\beta$, $\gamma$, ...) of the physical properties. Therefore, it is considered that optimization of the objective function expression with the weighting coefficients set as appropriate makes it possible to optimize the physical properties depending on intended use of a mixture, and therefore makes it possible to search for kinds of materials in the mixture and the component ratio thereof (mixture ratio).

In the optimization of the above objective function expression, searching for a combination of kinds of materials and a component ratio thereof so as to, for example, minimize the value of an objective function expression E may be considered as a combinatorial optimization problem. The combinatorial optimization problem is a problem of obtaining an optimum combination from a large number of combinations in consideration of various conditions and constraints.

Therefore, as a technique capable of solving the combinatorial optimization problem at high speed, a technique of performing calculation by an annealing method (annealing) using an annealing machine or the like has been proposed. This method is capable of searching for a solution of a combinatorial optimization problem in a short time by, for example, searching for a combination of variables (parameters) which minimize the value of an objective function expression by using an annealing machine or the like.

As described above, for example, if an objective function expression containing physical properties of a mixture as parameters is defined appropriately, the physical properties depending on intended use of the mixture may be optimized efficiently.

Here, the term representing a physical property value such as [physical property 1] in the above objective function expression is a term indicating the physical property of the mixture (mixture physical property) as described above, and is obtained in the related art by using a mathematical expression capable of estimating a physical property in a mixed state (physical property estimating equation) based on the values of the physical property of the respective materials. As the physical property estimating equation, for example, it is possible to use an equation for estimating a certain physical property value of a mixture by using the physical property values of respective materials for the certain physical property value to be estimated and a molar ratio (mixture molar ratio) of the materials in the mixture.

For example, the thermal conductivity and the viscosity of a mixture may be estimated by using the following physical property estimating equations as described in Shuzo Ohe, Physical Property Estimation Method (Japanese), Data Book Shuppan-sha and the like.

First, the thermal conductivity ($\lambda_{Lm}$) of a mixed refrigerant may be represented by the following equation.

$$\lambda_{Lm} = \sum_{i=1}^{N} \sum_{j=1}^{N} \phi_i \phi_j \lambda_{Lij} \qquad (1)$$

Here, "$\lambda_{Lij}$" and "$\varphi_i$" in the above equation (1) are represented by the following two equations.

$$\lambda_{Lij} = 2 \left( \frac{1}{\lambda_{Li}} + \frac{1}{\lambda_{Lj}} \right)^{-1} \qquad (2)$$

$$\phi_i = \frac{x_i V_i}{\sum_{j=1}^{N} x_j V_j} \qquad (3)$$

In the above equations, "$x_i$" denotes the molar fraction of an i-th component, "$\varphi_i$" denotes the volume fraction of the i-th component, and "$V$" denotes the molecular volume of the i-th component. For example, when N = 2, the above equation (1) enables estimation of the thermal conductivity of a mixture of two components as presented by the following equation.

$$\lambda_{Lm} = \phi_1^2 \lambda_{L1} + 2\phi_1 \phi_2 \lambda_{12} + \phi_2^2 \lambda_{L2} \qquad (4)$$

Kinematic viscosity ($v_m$) as the viscosity of a liquid mixture of two components may be estimated by the following equation.

$$v_m = \phi_1 v_1 e^{\phi_2 \alpha_2} + \phi_2 v_2 e^{\phi_1 \alpha_1} \qquad (5)$$

In the above equation, "$v_i$" denotes the kinematic viscosity of an i-th component, "$\varphi_i$" denotes the volume fraction of the i-th component, and $\alpha_1$ and $\alpha_2$ are expressed by the following two equations, respectively, where "$v_1 < v_2$" is satisfied.

$$\alpha_1 = -1.7 \ln \left( \frac{v_2}{v_1} \right) \qquad (6)$$

$$\alpha_2 = 0.27 \ln \left( \frac{v_2}{v_1} \right) + \left( 1.3 \ln \left( \frac{v_2}{v_1} \right) \right)^{\frac{1}{2}} \qquad (7)$$

As in the examples described above, regarding a physical property (performance) for which a theoretical or empirical physical property estimating equation is known, it is possible to estimate the physical property of a mixture based on the values of the physical property of respective materials and the mixture molar ratio thereof.

However, in an attempt to predict a physical property for which a physical property estimating equation does not exist, the related art using a physical property estimating equation has no way to define a term that represents the physical property value such as [physical property 1] in the above objective function expression. Therefore, in an attempt to predict a physical property for which a physical property estimating equation does not exist, the related art using a physical property estimating equation has difficulty in constructing an objective function expression for optimizing the physical property of a mixture, and accordingly has difficulty in predicting the physical property of a mixture. As described above, in an attempt to predict a physical property for which a physical property estimating equation does not exist, there is a problem that the related art using a physical property estimating equation has no way to predict and identify the physical property of a mixture and therefore fails to optimize the physical property of the mixture.

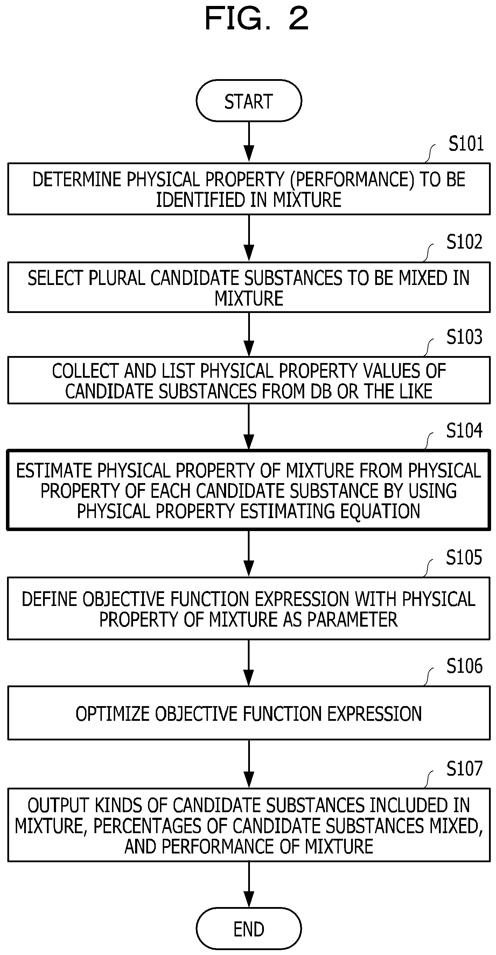

Here, a sequence and others of a technique using a physical property estimating equation in order to obtain a physical property of a mixture will be described with reference to a flowchart illustrated in FIG. 2. First, in the technique of obtaining a physical property of a mixture by using a physical property estimating equation, for example, a physical property (performance) to be identified in the mixture is determined (S101). Next, in this technique, for example, a plurality of candidate substances to be mixed in the mixture are selected (S102).

Subsequently, in this technique, for example, physical property values of the candidate substances are collected from a database (DB) or the like and listed (S103). In the technique of obtaining a physical property of a mixture by using a physical property estimating equation, the physical property of the mixture is estimated from the values of the physical property of the respective candidate substances by using, for example, a physical property estimating equation (S104). Next, in this technique, for example, an objective function expression having the physical property of the mixture as a parameter is defined (S105). Subsequently, in this technique, for example, the objective function expression is optimized (S106). Next, in this technique, for example, the kinds of the candidate substances included in the mixture, the percentages of the candidate substances mixed, and the physical property (physical property value) of the mixture are output, and the process is ended (S107).

For example, as illustrated in FIG. 2, in the technique of obtaining a physical property of a mixture by using a physical property estimating equation, a predetermined physical property of a mixture in an objective function expression for identifying the physical property (performance) of the mixture is estimated by using the physical property estimating equation. Therefore, in an attempt to predict a physical property for which a physical property estimating equation does not exist, this technique has no way to define the objective function expression, and therefore is incapable of predicting and identifying the physical property of the mixture.

As described above, for a mixture such as a vulcanized rubber composition or a melt obtained by casting, there has been proposed the technique of optimizing a physical property of the mixture by predicting the physical property of the mixture using machine learning and determining the composition (component contents) of materials in the mixture.

However, in this related art, the prediction accuracy of a physical property of a mixture may become insufficient in some cases such as a case where, for example, learning datasets for use in the machine learning are insufficient. This related art is to predict a physical property of a mixture by using a module (model) obtained by machine learning using datasets for learning prepared in advance, and is incapable of evaluating the prediction accuracy of the module (model), updating the module (model), and doing the like. Therefore, there is a problem that this related art has difficulty in improving the prediction accuracy of a physical property of a mixture.

As described above, the related art has difficulty in predicting a physical property (performance) for which a mathematical expression capable of estimating a physical property in a mixed state (physical property estimating equation) does not exist. In a case of using machine learning, the related art has problems that the prediction accuracy of a physical property of a mixture is insufficient in some cases, and that it is difficult to improve the prediction accuracy of a physical property of a mixture even when the prediction accuracy is insufficient.

Therefore, the present inventor has made extensive studies on a method and the like capable of predicting and identifying a physical property of a mixture with high accuracy even in the case of predicting the physical property for which a mathematical expression capable of estimating a physical property in a mixed state (physical property estimating equation) does not exist, and has obtained the following findings.

For example, the present inventor has found that the following mixture physical property identification method and the like are capable of predicting and identifying a physical property of a mixture with high accuracy even in a case of predicting the physical property for which a mathematical expression capable of estimating a physical property in a mixed state (physical property estimating equation) does not exist.

A mixture physical property identification method as an example of the technique disclosed herein includes: a step of creating a prediction term for predicting at least one physical property of a mixture of a plurality of candidate substances; and a step of identifying the physical property of the mixture by using an objective function expression including the prediction term; in which the step of creating a prediction term includes a step of obtaining a dataset indicating a physical property of each of a plurality of mixtures each containing two or more candidate substances among a plurality of candidate substances, and a step of setting at least some of the datasets indicating the physical property as first learning datasets, and comparing the first learning datasets with corresponding datasets corresponding to the first learning datasets in a first prediction model based on the first learning datasets; when the first learning datasets and the corresponding datasets demonstrate a predetermined correlation, the prediction term is created based on regression coefficients of the respective candidate substances obtained from the first prediction model, when the first learning datasets and the corresponding datasets do not demonstrate the predetermined correlation, the step of creating a prediction term further includes a step of obtaining virtual datasets based on an integration model obtained by integrating a plurality of prediction models generated based on the datasets indicating the physical property, and a step of setting at least some of the virtual datasets as second learning datasets, and comparing the first learning datasets with corresponding datasets corresponding to the first learning datasets in a second prediction model based on the second learning datasets, when the first learning datasets and the corresponding datasets demonstrate the predetermined correlation, the prediction term is created based on regression coefficients of the respective candidate substances obtained from the second prediction model.

In the example of the technique disclosed herein, a dataset indicating a physical property (physical property value dataset) is obtained for each mixture among mixtures each containing two or more of candidate substances, and the regression coefficients of the respective candidate substances are obtained by way of a prediction model based on the datasets indicating the physical property, thereby creating a prediction term for predicting the physical property of the mixture.

The dataset indicating the physical property (physical property value dataset) of each mixture may be obtained, for example, based on an actual experiment, calculation (physical property simulation), or the like for the mixture containing two or more of the candidate substances. As described above, in the example of the technique disclosed herein, for example, datasets on a physical property (physical property value datasets) are obtained for a plurality of kinds of mixtures, and are used for learning or evaluation of a prediction model.

In the example of the technique disclosed herein, at least some of the datasets indicating the physical property are set as the first learning datasets, and a "first prediction model" based on the first learning datasets is created. For example, in the example of the technique disclosed herein, the datasets indicating the physical property are divided for use into prediction model verification datasets to be used for verification of a prediction model and first learning datasets to be used for learning of the prediction model, which are then used for verification and for learning of a first prediction model, respectively.

As described above, in the example of the technique disclosed herein, a first prediction model for predicting one physical property of a mixture is creased by using, as the learning datasets, the datasets indicating the physical property calculated from an actual experiment, a physical property simulation, or the like.

In the example of the technique disclosed herein, each prediction value in the first prediction model is compared with a first learning dataset corresponding to the prediction value to obtain a correlation between the prediction values and the first learning datasets.

In the example of the technique disclosed herein, for example, the prediction accuracy of the first prediction model is evaluated by obtaining a correlation (degree of correlation) between prediction values of the physical property predicted by using the first prediction model and the first learning datasets corresponding to the respective prediction values.

Next, in the example of the technique disclosed herein, when the prediction values and the first learning datasets demonstrate a predetermined correlation (when the prediction accuracy of the first prediction model is sufficient), the regression coefficients of the respective candidate substances are obtained according to the first prediction model to create a prediction term.

As described above, in the example of the technique disclosed herein, when the prediction accuracy of the first prediction model is considered to be sufficient, the prediction term for predicting at least one physical property of a mixture of a plurality of candidate substances is created according to the first prediction model. In this case, since the prediction term created according to the first prediction model has sufficient prediction accuracy, it is possible to predict and identify the physical property of the mixture with high accuracy by identifying the physical property of the mixture using the objective function expression including this prediction term without using a physical property estimating equation.

On the other hand, when the prediction values of the first prediction model and the first learning datasets do not demonstrate the predetermined correlation, a plurality of prediction models (composition-by-composition prediction models) are prepared based on the datasets indicating the physical property in the example of the technique disclosed herein. For example, when the prediction accuracy of the first prediction model is insufficient, a prediction model for each type of combinations of candidate substances (materials) is prepared by using the datasets on the physical property in the example of the technique disclosed herein. The prediction model herein is created to be capable of predicting a physical property value that the combination may take along with a change in the component ratio (mixture ratio).

In the example of the technique disclosed herein, virtual datasets are obtained (created) based on an integration model in which the plurality of prediction models thus prepared are integrated together. For example, in the example of the technique disclosed herein, the integration model in which the plurality of prediction models are integrated together is created based on the plurality of prediction models prepared, and the virtual datasets are created based on the created integration model.

The integration model in which the plurality of prediction models are integrated together may be created, for example, in such a way that composition-by-composition prediction models of a plurality of kinds of mixtures (distribution curves of the physical property values in the respective compositions) are created based on distributions of the datasets on the physical property and these composition-by-composition prediction models are integrated together. The integration model may be, for example, a "Gaussian mixture model" based on the plurality of prepared prediction models.

In the example of the technique disclosed herein, virtual datasets are created based on the integration model created in this manner, which makes it possible to expand the distribution of datasets on the physical property calculated from an actual experiment, a physical property simulation, or the like, and increase datasets usable for learning. For example, in the example of the technique disclosed herein, it is possible to increase the number of datasets usable to create the prediction model by creating and preparing the virtual datasets based on the integration model, and thus to improve the prediction accuracy of the prediction model.

Next, in the example of the technique disclosed herein, a "second prediction model" according to the integration model is created by using at least some of the virtual datasets as second learning datasets. For example, in the example of the technique disclosed herein, the second prediction model is created by using, as the second learning datasets, some of the virtual datasets created based on the integration model.

In the example of the technique disclosed herein, the first learning datasets are compared with corresponding datasets (prediction values) corresponding to the first learning datasets in the second prediction model to obtain a correlation between the first learning datasets and the prediction values. In the example of the technique disclosed herein, for example, the prediction accuracy of the second prediction model is evaluated by obtaining the correlation between the prediction values predicted using the second prediction model and the first learning datasets corresponding to the prediction values.

Subsequently, in the example of the technique disclosed herein, when the first learning datasets and the prediction values obtained by the second prediction model demonstrate the predetermined correlation, a prediction term is created by obtaining the regression coefficients of the respective candidate substances according to the second prediction model. For example, in the example of the technique disclosed herein, when the prediction accuracy of the second prediction model is considered to be sufficient, a prediction term for predicting at least one physical property of a mixture of a plurality of candidate substances is created according to the second prediction model.

In this case, since the prediction term created according to the second prediction model has sufficient prediction accuracy, it is possible to predict and identify the physical property of the mixture with high accuracy by identifying the physical property of the mixture using the objective function expression including this prediction term without using a physical property estimating equation.

In the example of the technique disclosed herein, for example, it is preferable that the creation of the virtual datasets and the creation of the second prediction model be repeated until the correlation of the second prediction model with the learning datasets has the predetermined correlation. This enables further improvement of the prediction accuracy of the second prediction model, and accordingly leads to the higher prediction accuracy of the physical property of the mixture using the objective function expression including the prediction term based on the second prediction model.

As described above, in the example of the technique disclosed herein, for example, a prediction model is created by using datasets indicating a physical property (dataset on the physical property, physical property value datasets) of each of mixtures. Then, depending on the prediction accuracy of the prediction model, virtual datasets are generated according to an integration model in which the distributions of the datasets indicating the physical property are integrated together. In the example of the technique disclosed herein, for example, the number of datasets usable to create the prediction model may be increased by the generated virtual datasets, and thus the prediction accuracy of the prediction model (second prediction model) may be improved.

In the example of the technique disclosed herein, for example, the accuracy of a prediction model is evaluated based on a correlation between prediction values obtained by the prediction model and learning datasets corresponding to the prediction values. This makes it possible to create the prediction term based on the prediction model having sufficient prediction accuracy. Thus, in the example of the technique disclosed herein, it is possible to identify the physical property of the mixture by using the objective function expression including the prediction term with the sufficient prediction accuracy, and is therefore possible to further increase the prediction accuracy of the physical property of the mixture.

As discussed above, the technique disclosed herein does not have to use a mathematical expression capable of estimating a physical property in a mixed state (physical property estimating equation) when creating a prediction term, and is capable of predicting and identifying the physical property of a mixture with high accuracy even in a case of predicting the physical property for which a physical property estimating equation does not exist.

Hereinafter, steps included in a mixture physical property identification method disclosed herein will be described in detail with reference to the drawings.

The mixture physical property identification method disclosed herein includes at least a step of creating a prediction term and a step of identifying a physical property, and further includes other steps as requested.

<Mixture>

A mixture of which physical properties are to be identified in the technique disclosed herein is not particularly limited as long as it is a mixture of a plurality of candidate substances and may be appropriately selected in accordance with the intended purpose. For example, in the technique disclosed herein, any mixture may be appropriately selected depending on the intended purpose without particular limitation, as long as the mixture may be changed in various physical properties and characteristics when the kinds and amounts of substances mixed therein are changed.

In the technique disclosed herein, candidate substances (materials) to be mixed in a mixture are not particularly limited, and may be appropriately selected in accordance with the intended purpose. The number of kinds of candidate substances mixed in a mixture may be any number more than one (two or more) without particular limitation and be appropriately selected in accordance with the intended purpose.

In the example of the technique disclosed herein, it is preferable that candidate substances (materials) to be mixed in a mixture be selected according to the type of the mixture, for example, from a database in which physical properties and other data of many substances are recorded.

In the technique disclosed herein, the physical properties of a mixture to be identified are not particularly limited, and may be appropriately selected in accordance with the intended purpose. The physical properties of a mixture to be identified by the technique disclosed herein may be selected depending on the physical properties requested for the mixture, for example, according to the type of the mixture.

Examples of a mixture of which physical properties are to be identified in the technique disclosed herein include a refrigerant, a detergent, a food, and so forth.

The refrigerant is not particularly limited as long as it is a refrigerant (mixed refrigerant) in which a plurality of candidate substances (materials) are mixed, and may be appropriately selected in accordance with the intended purpose. The refrigerant may be in the form of a gas at room temperature or in the form of a liquid at room temperature.

Examples of the physical properties of the mixed refrigerant include thermal resistance, thermal conductivity, specific heat, viscosity, vapor pressure, boiling point, surface tension, latent heat of vaporization, combustibility, flammability, ignitability, toxicity, energy efficiency, environmental influence, and so on. The energy efficiency may be expressed by using, for example, the coefficient of performance (COP) or the like. Examples of the environmental influence include a global warming potential (GWP), an ozone-depleting potential (ODP), and so on.

The detergent is not particularly limited as long as it is a detergent in which a plurality of candidate substances (materials) are mixed, and may be appropriately selected in accordance with the intended purpose. Examples of the detergent include an aqueous detergent, a semi-aqueous detergent, a hydrocarbon-based detergent, an alcohol-based detergent, a chlorine-based detergent, a fluorine-based detergent, a bromine-based detergent, and so on.

The physical properties of the detergent are not particularly limited, and may be appropriately selected in accordance with the intended purpose. Examples of the physical properties of the detergent include specific heat, viscosity, surface tension, latent heat of vaporization, combustibility, flammability, toxicity, hydrogen ion exponent (pH), evaporation rate, permeability, detergency for a specific target, storage stability, and so on.

The food is not particularly limited as long as it is a food in which a plurality of candidate substances (materials) are mixed, and may be appropriately selected in accordance with the intended purpose. Examples of the food include coffee and so on. When the mixture of which physical properties are to be identified is coffee, for example, kinds of coffee beans to be raw materials of the coffee and the amounts of the coffee beans are determined in the example of the technique disclosed herein. For example, in the example of the technique disclosed herein, it is possible to determine an appropriate blending ratio of the coffee beans in so-called blended coffee.

The physical properties (taste characteristics) of the coffee are not particularly limited and may be appropriately selected in accordance with the intended purpose. Examples of the physical properties of the coffee include aroma, acidity, bitterness, body, and so on.

<Objective Function Expression>

As described above, in the example of the technique disclosed herein, it is possible to use an objective function expression which includes a prediction term for predicting at least one physical property of a mixture of a plurality of candidate substances, and which is capable of identifying the physical property of the mixture. The prediction term is created based on the regression coefficients of respective candidate substances obtained from the first prediction model or the second prediction model.

The objective function expression may be selected as appropriate depending on a physical property (performance) of a mixture, a constraint imposed on selection of substances to be mixed in the mixture, and so on. As the objective function expression, for example, it is possible to use an expression which includes values of physical properties of a mixture as variables and takes a minimum value when the mixture contains an optimum combination of substances. Therefore, it is possible to optimize the physical properties of the mixture by obtaining a combination of the variables with which the objective function expression takes the minimum value.

In the example of the technique disclosed herein, the objective function expression represented by the following expression may be preferably used.

$$E = \alpha \cdot [\text{Mixture Physical Property Prediction 1}] + \beta \cdot [\text{Mixture Physical Property Prediction 2}] + \gamma \cdot [\text{Mixture Physical Property Prediction 3}] + ... + \text{Constraint Term},$$

where E is an objective function expression and $\alpha$, $\beta$ and $\gamma$ are weighting coefficients. The constraint term is a term that represents a constraint such as the number of selected materials (substances) in the objective function expression. In addition, "..." in the above objective function expression means that the objective function expression may include, as appropriate, physical properties other than "Mixture Physical Property Prediction 1", "Mixture Physical Property Prediction 2", and "Mixture Physical Property Prediction 3", and weighting coefficients other than $\alpha$, $\beta$, and $\gamma$.

Here, each of "Mixture Physical Property Prediction 1" to "Mixture Physical Property Prediction 3" in the objective function expression denotes a prediction term for predicting a physical property (mixture property) of the mixture. For example, it is possible to use an objective function expression that includes a plurality of prediction terms for predicting physical properties (performance) of a mixture and further includes a constraint term that represents a constraint in the objective function expression in the example of the technique disclosed herein.

In the above objective function expression, each term (prediction term) of "Mixture Physical Property Prediction" is created by obtaining the regression coefficients of the respective candidate substances using the prediction model (the first prediction model or the second prediction model). Therefore, "Mixture Physical Property Prediction" in the above objective function expression includes, for example, the regression coefficients of the respective candidate substances, the component ratio of the candidate substances, and a constant term.

For example, "Mixture Physical Property Prediction" in the above objective function expression may preferably use one represented by the following expression:

$$[\text{Mixture Physical Property Prediction}] = a \cdot [\text{Component Ratio of Candidate Substance A}] + b \cdot [\text{Component Ratio of Candidate Substance B}] + c \cdot [\text{Component Ratio of Candidate Substance C}] + ... + \text{Constant Term, where E is an objective function expression and a, b, and c are regression coefficients.}$$

In the example of the technique disclosed herein, all the terms that represent the mixture physical properties of the mixture in the objective function expression do not have to be created based on the regression coefficients of the respective candidate substances obtained from the first prediction model or the second prediction model, and the objective function expression may include a prediction term created by any other method.

Examples appropriately usable as the prediction term created by the other method include a prediction term using the aforementioned mathematical expression capable of estimating a physical property in a mixed state (physical property estimating equation), a prediction term using a weighted mean of the physical property values of substances to be mixed based on the molar concentrations of the respective substances, and so on. As a physical property of each substance for use to create a prediction term in any of these other methods, it is possible to use, for example, a literature value, an actual measurement value (a value obtained by actually performing an experiment), a value calculated based on a physical property simulation, or the like.

As the physical property estimating equation, a theoretical or empirical physical property estimating equation based on the physical property of candidate substances may be appropriately selected and used as described above, and the equations disclosed in literature such as "Physical Property Estimation Method (Japanese) (Shuzo Ohe, Data Book Shuppan-sha)" or the like may be used.

As a prediction term using a weighted mean of the physical property values of substances to be mixed based on the molar concentrations of the respective substances, for example, a prediction term obtained as follows may be used.

For example, a case of obtaining (estimating) the specific heat of a mixture will be described by using an example in which 100 mol of a mixture contains 50 mol of a substance A, 30 mol of a substance B, and 20 mol of a substance C. In this example, the specific heat of the substance A is 2000 J/(kg·K), the specific heat of the substance B is 4000 J/(kg·K), and the specific heat of the substance C is 1000 J/(kg·K). Under these conditions, the specific heat of the mixture is obtained by using the values of the specific heat of the respective substances based on the molar concentrations of the respective substances, for example, as presented in the following equation.

$$\text{Specific Heat of Mixture} = 2000 \times (50/100) + 4000 \times (30/100) +$$

$$1000 \times (20/100) = 2400 \text{ J/(kg·K)}$$

As described above, in the example of the technique disclosed herein, for example, a value of a weighted mean of the physical property values of substances to be mixed in a mixture based on the molar concentrations of the respective substances may be used as the physical property of the mixture.

It is preferable that the constraint term in the objective function expression include at least one of the following four constraints: A constraint that the number of kinds of candidate substances mixed in a mixture is a predetermined number; A constraint that a total of the percentages of candidate substances mixed in the mixture is 100%; A constraint that the same substance is not selected two or more times as a candidate substance to be mixed in the mixture; and A constraint that the mixture contains a predetermined candidate substance.

First, the "constraint that the number of kinds of candidate substances mixed in a mixture is a predetermined number" among the above four constraints will be described.

In optimization of the physical properties of a mixture, there is a case where the number of candidate substances to be mixed is set in advance and then candidate substances to be mixed in the mixture are searched for. When the above-listed "constraint that the number of kinds of candidate substances mixed in a mixture is a predetermined number" is imposed on such a case, it is possible to narrow down the search to mixtures in each of which the preset predetermined number of candidate substances are mixed.

The "constraint that the number of kinds of candidate substances mixed in a mixture is a predetermined number" may be, for example, a penalty term that increases the value of the objective function expression when the mixture is composed of a combination in which the number of kinds of candidate substances mixed is not the predetermined number.

Next, the "constraint that a total of the percentages of candidate substances mixed in the mixture is 100%" among the above four constraints will be described.

In the search for a combination of substances to be mixed in a mixture of a plurality of candidate substances, the total of the percentages (contents) of the candidate substances mixed with respect to the total amount of the mixture is usually 100%. Therefore, when the above-listed "constraint that a total of the percentages of candidate substances mixed in the mixture is 100%" is imposed, it is possible to narrow down the search to mixtures in each of which the total of the percentages of candidate substances mixed is 100%.

The "constraint that a total of the percentages of candidate substances mixed in the mixture is 100%" may be, for example, a penalty term that increases the value of the objective function expression when the mixture is composed of a combination in which the total of the percentages of the candidate substances mixed is not 100%.

Next, the "constraint that the same substance is not selected two or more times as a candidate substance to be mixed in the mixture" among the above four constraints will be described.

In the search for a combination of candidate substances to be mixed in a mixture of a plurality of candidate substances, the search for combinations each including various candidate substances might fail if combinations in each of which the same candidate substance is selected two or more times were searched. Therefore, when the above-listed "constraint that the same substance is not selected two or more times as a candidate substance to be mixed in the mixture" is imposed, it is possible to narrow down the search to mixtures each composed of a combination of different candidate substances.

The "constraint that the same substance is not selected two or more times as a candidate substance to be mixed in the mixture" may be, for example, a penalty term that increases the value of the objective function expression when the mixture is composed of a combination in which the same candidate substance is selected two or more times as a candidate substance to be mixed.

Next, the "constraint that the mixture contains a predetermined candidate substance" among the above four constraints will be described.

In the search for a combination of candidate substances to be mixed in a mixture of a plurality of candidate substances, there is a case where a candidate substance to be a base of the mixture is set in advance, and candidate substances to be mixed in the mixture are searched out so as to include the substance to be the base. Therefore, when the above-listed "constraint that the mixture contains a predetermined candidate substance" is imposed, it is possible to narrow down the search to mixtures each containing the candidate substance set as the base in advance.

The "constraint that the mixture contains a predetermined candidate substance" may be, for example, a penalty term that increases the value of the objective function expression when the mixture is composed of a combination not containing the predetermined candidate substance.

<Creation of Prediction Term (Step of Creating Prediction Term)>

Here, in creating an objective function expression in the example of the technique disclosed herein, a plurality of mixtures each containing two or more of candidate substances are prepared, a dataset indicating a physical property of each of all the mixtures is obtained, and at least some of the datasets indicating the physical property is set as first learning datasets.

As described above, the dataset indicating the physical property (physical property value dataset) of each of all the mixtures may be obtained, for example, based on an actual experiment, calculation (physical property simulation), or the like for the mixtures each containing two or more of the candidate substances. In obtaining the datasets indicating the physical property, for example, it is preferable to select a combination of mixtures in which each of all the candidate substances for the mixtures is used at least once.

The physical property simulation is not particularly limited as long as it is capable of obtaining the datasets indicating the physical property (physical property value datasets) of the mixtures, and may be appropriately selected in accordance with the intended purpose. For example, a molecular dynamics simulation (molecular dynamics calculation) may be used.

The molecular dynamics (MD) simulation may be performed by using a known program (software). By performing the molecular dynamics simulation, for example, datasets on a physical property such as thermal conductivity may be obtained.

<<First Prediction Model>>

In the example of the technique disclosed herein, at least some of datasets indicating a physical property are set as first learning datasets and a "first prediction model" based on the first learning datasets is created as described above. A percentage of datasets selected as the first learning datasets from the datasets indicating the physical property is preferably half or more of the total number of the datasets indicating the physical property, and may be, for example, about 80%.

In the example of the technique disclosed herein, for example, the datasets indicating the physical property may be divided into prediction model verification datasets to be used for verification of a prediction model and first learning datasets to be used for learning of the prediction model, which may be then used for verification and for learning of a first prediction model, respectively.

In the example of the technique disclosed herein, prediction values (corresponding datasets) in the first prediction model are compared with the first learning datasets corresponding to the prediction values to obtain a correlation between the prediction values and the first learning datasets. Next, in the example of the technique disclosed herein, in a case where the prediction values (corresponding datasets) and the first learning datasets demonstrate a predetermined correlation, a prediction term is created by obtaining the regression coefficients of the respective candidate substances according to the first prediction model.

The predetermined correlation between the first learning datasets and the corresponding datasets is not particularly limited as long as it may be used as an index for evaluating the prediction accuracy of the first prediction model, and may be appropriately selected in accordance with the intended purpose. As the predetermined correlation between the first learning datasets and the corresponding datasets, it is preferable to use a correlation in which, for example, a mean absolute error (MAE) and a root mean square error (RMSE) are considered.

For example, it is preferable that the predetermined correlation between the first learning datasets and the corresponding datasets be "RMSE/MAE (the ratio of the root mean square error to the mean absolute error)". In the example of the technique disclosed herein, for example, it is preferable that a prediction model with which the value of "RMSE/MAE" is within a predetermined range be evaluated as a prediction model with high prediction accuracy.

The reason why it is preferable to use "RMSE/MAE" for evaluation of the prediction model, rather than an index such as $r^2$ (coefficient of determination) (alone), RMSE (alone), or MAE (alone) will be described later in Example.

In the evaluation of the prediction model by using "RMSE/MAE", a value of "RMSE/MAE" for evaluating a prediction model as having high prediction accuracy may be, for example, a value around "1.253".

The reason why it is possible to evaluate that the accuracy of the prediction model is high when the value of "RMSE/MAE" is around "1.253" will be described below.

First, RMSE and MAE are expressed by the following equations, respectively:

$$RMSE = \sqrt{\frac{1}{N}\sum_{i=1}^{N}(y_i - y_p)^2} \qquad (8)$$

$$MAE = \frac{1}{N}\sum_{i=1}^{N}|y_i - y_p| \qquad (9)$$

where $y_i$ denotes a dataset on the physical property (actual correct value) obtained by a physical property simulation or the like, $y_p$ denotes a prediction value (corresponding dataset corresponding to the dataset on the physical property) calculated by using a prediction model constructed based on learning datasets, and N denotes the number of the datasets.

In each of RMSE and MAE, the closer to "0 (zero)" the value, the smaller an estimation error (prediction error).

When $e_i$ denotes the absolute value of an error of the prediction value $y_p$ with respect to the dataset on the physical property (actual correct value) $y_i$, the second power of RMSE ($RMSE^2$) and the second power of MAE ($MAE^2$) are expressed by the following equations derived from the above equations (8) and (9).

$$RMSE^2 = \frac{1}{N}\sum_{i=1}^{N}e_i^2 \qquad (10)$$

$$MAE^2 = \frac{1}{N^2}\left(\sum_{i=0}^{N}e_i\right)^2 \qquad (11)$$

Here, the variance $Var(e_i)$ is expressed by the following equation using a difference between "the mean of the second power" and "the second power of the mean".

$$RMSE^2 - MAE^2 = Var(e_i) \qquad (12)$$

Here, MAE is nothing more than the mean $MEAN(e_i)$ of $e_i$. Thus, by converting the above equation (12), the ratio of RMSE to MAE is expressed by the following equation.

$$\frac{RMSE}{MAE} = \sqrt{1 + \frac{Var(e_i)}{MEAN(e_i)^2}} \qquad (13)$$

When the error is 0 and follows the normal distribution of the standard deviation $\sigma$, the distribution of the absolute value $e_i$ ($\geq 0$) of the error is the distribution of the absolute value of the normal distribution. Thus, a probability density function f is expressed by the following equation.

$$f = 2 \times \frac{1}{\sqrt{2\pi}\sigma}\exp\left(-\frac{e^2}{2\sigma^2}\right) \qquad (14)$$

Therefore, using the above equation (14), MEAN(e) and Var(e) are expressed by the following equations.

$$MEAN(e) = \int_0^\infty e \times \frac{2}{\sqrt{2\pi}\sigma} \exp\left(-\frac{e^2}{2\sigma^2}\right) de = \sqrt{\frac{2}{\pi}}\sigma \qquad (15)$$

$$Var(e) = \int_0^\infty \left(e - MEAN(e)\right)^2 \times \frac{2}{\sqrt{2\pi}\sigma} \exp\left(-\frac{e^2}{2\sigma^2}\right) de = \left(1 - \frac{2}{\pi}\right)\sigma^2 \quad (16)$$

Therefore, when the above equations (15) and (16) are substituted into the above equation (13), the following equation is obtained.

$$\frac{RMSE}{MAE} = \sqrt{\frac{\pi}{2}} \fallingdotseq 1.253 \qquad (17)$$

From the above, when the prediction model sufficiently represents the feature of the datasets on the physical property (actual correct values), the ratio of RMSE to MAE is a value around 1.253. In this case, only noise as following the normal distribution remains as an error.

For example, for the reason described above, when the value of "RMSE/MAE" is around "1.253", the prediction model may be evaluated as having high accuracy in the example of the technique disclosed herein.

It is preferable that a value around "1.253" in "RMSE/MAE" be set to, for example, "1.253 $\pm$ 0.03". For example, in the example of the technique disclosed herein, it is preferable that a prediction model with which "RMSE/MAE" is "1.253 $\pm$ 0.03" be determined as demonstrating the predetermined correlation and evaluated as a prediction model with high prediction accuracy.

For example, in the example of the technique disclosed herein, the predetermined correlation is preferably set such that the ratio of the root mean square error (RMSE) to the mean absolute error (MAE) with respect to at least either the first learning datasets or the second learning datasets is 1.253 $\pm$ 0.03. In this way, in the example of the technique disclosed herein, it is possible to more clearly evaluate the accuracy of the prediction model, and to create a prediction term based on the more reliable prediction model.

In the example of the technique disclosed herein, it is preferable that the prediction models (the first prediction model and the second prediction model) be derived by performing multiple regression (multivariate analysis) based on learning datasets. For example, in the example of the technique disclosed herein, it is preferable that at least one of the first prediction model and the second prediction model be derived by a multiple regression equation based on the first learning datasets or the second learning datasets. The multiple regression analysis means a regression analysis, which is a type of multivariate analysis, using two or more explanatory variables, and is an analysis method capable of obtaining a correlation between the two or more explanatory variables and one objective function. The form or the like of the explanatory variables is not particularly limited, and may be appropriately selected in accordance with the intended purpose. The form or the like of the explanatory variables is not limited to a one-dimensional (linear) form, but a nonlinear term may be present.

In the multiple regression, for example, when the prediction values predicted by using the prediction model are plotted along the vertical axis and the actual physical property values (learning datasets) are plotted along the horizontal axis, the more plots on a straight line obtained by the multiple regression, the higher the accuracy of the prediction model. Since a result of optimization using a prediction model is influenced by the number of explanatory variables (the number of kinds of candidate substances) used for prediction, it is more important to enhance the accuracy of the prediction model as the number of explanatory variables increases.

In the example of the technique disclosed herein, when a prediction term is created by obtaining the regression coefficients of the respective candidate substances from the created prediction model, it is possible to easily calculate the regression coefficients of the respective candidate substances by processing information on the prediction model (such as plot data of prediction values and actual physical property values) using a Python library or doing the like.

<<Second Prediction Model>>

Here, in the example of the technique disclosed herein, when the prediction values of the first prediction model and the first learning datasets do not demonstrate the predetermined correlation (the prediction accuracy of the first prediction model is insufficient), a plurality of prediction models are prepared based on the datasets indicating the physical property as described above.

The plurality of prediction models prepared may be composition-by-composition prediction models. For example, it is preferable to prepare, for each type of combinations of candidate substances (materials), a prediction model capable of predicting a physical property value that the combination may take along with a change in the component ratio (mixture ratio).

In the example of the technique disclosed herein, virtual datasets are obtained (created) based on an integration model in which the plurality of prediction models thus prepared are integrated together. As described above, the integration model obtained by integrating the plurality of prediction models may be the "Gaussian mixture model" based on the plurality of prepared prediction models.

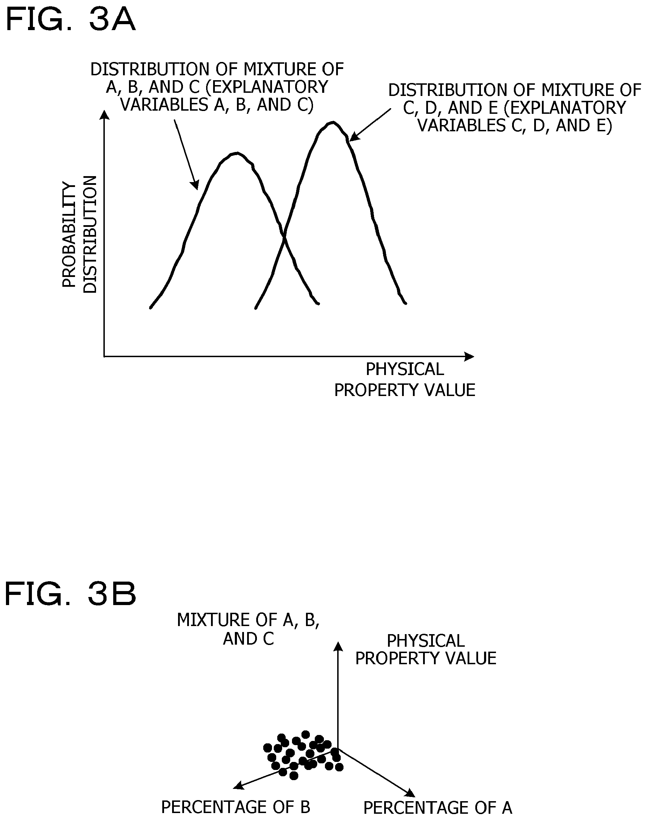

FIG. 3A is a diagram illustrating an example of composition-by-composition prediction models of a plurality of kinds of mixtures based on the distributions of the datasets on the physical property (physical property value datasets). The example illustrated in FIG. 3A illustrates an example in which prediction models are created in such a way that A, B, C, D, and E representing five kinds of materials (candidate substances) are used as explanatory variables and the physical property values obtained when three kinds A, B, and C are mixed and the physical property values obtained when three kinds C, D, and E are mixed are set as learning datasets. In the example of FIG. 3A, a normal distribution (Gaussian distribution) followed by the physical property values of a mixture of the three kinds A, B, and C mixed and a normal distribution followed by the physical property values of a mixture of the three kinds C, D, and E mixed are illustrated in an overlapping manner. In the example of FIG. 3A, the learning datasets are present on the lines of the normal distributions.

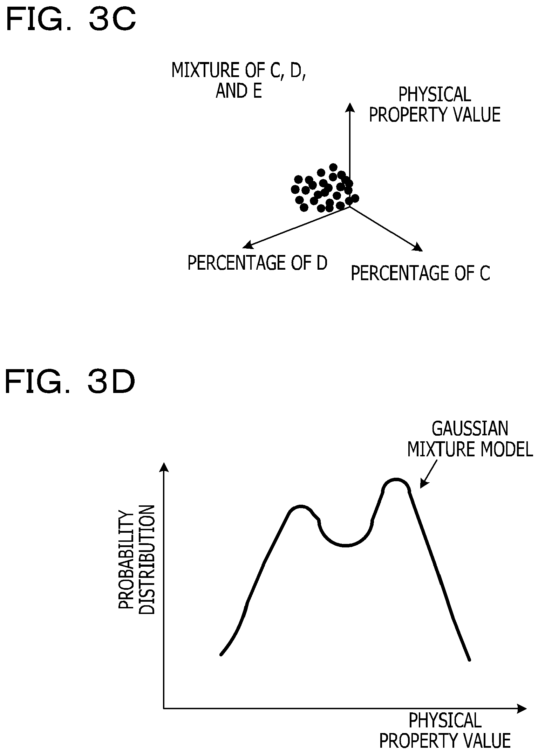

FIG. 3B is a diagram illustrating an example of a relationship among the physical property value of the mixture of A, B, and C, the percentage of A, and the percentage of B in FIG. 3A. Similarly, FIG. 3C is a diagram illustrating an example of a relationship among the physical property value of the mixture of C, D, and E, the percentage of C, and the percentage of D in FIG. 3A.

The example in FIG. 3B illustrates a distribution of the physical property values according to the percentage of A and the percentage of B in a case where the percentage of C is fixed. Similarly, the example in FIG. 3 C illustrates a distribution of the physical property values according to the percentage of C and the percentage of D in a case where the percentage of E is fixed.

As illustrated in FIG. 3B and FIG. 3C, in a case where a mixture is produced by selecting three kinds of materials from the five kinds of materials, a plurality of composition-by-composition prediction models for the respective types of combinations of three kinds of materials are each created by obtaining the distribution of the physical property values that the mixture may take along with a change in the component ratio (mixture ratio).

FIG. 3D is a diagram illustrating an example of a Gaussian mixture model in which the composition-by-composition prediction models of the plurality of kinds of mixtures illustrated in FIG. 3A are integrated and combined together. In the example of the technique disclosed herein, as illustrated in FIG. 3D, for example, it is possible to expand the distribution of datasets by a Gaussian mixture model representing the composition-by-composition prediction models (normal distributions each followed by the physical property values of a mixture) combined together.

Although the Gaussian mixture model is illustrated as a two-dimensional graph in FIG. 3D for convenience of explanation, the Gaussian mixture model is a multidimensional model corresponding to the number of explanatory variables in actual calculation.

In the example of the technique disclosed herein, a predetermined number of virtual datasets are generated according to an integration model such as a Gaussian mixture model. The generation of the virtual datasets according to the integration model may be done, for example, by generating datasets in which physical property values are randomly set so as to satisfy the probability distribution in the integration model. For example, in the example of the technique disclosed herein, the virtual datasets may be generated by virtually generating data points on the line of the distribution of the integration model.

In the example of the technique disclosed herein, it is possible to increase the number of datasets usable to create a prediction model by generating the virtual datasets in this manner, and thus improve the prediction accuracy of the prediction model.

The prediction accuracy of the prediction model (second prediction model) based on the generated virtual datasets depends on the number of the virtual datasets generated. For example, when the number of virtual datasets used for learning is too large, it may be difficult to improve the prediction accuracy of the prediction model because data points are also sampled from portions of the Gaussian mixture model where the density of the distribution is low (bottom portions of the distribution).

Therefore, in the example of the technique disclosed herein, it is preferable to control the number of virtual datasets generated so that the prediction accuracy of the second prediction model may be further improved.

As described above, in the example of the technique disclosed herein, the prediction accuracy of the prediction model may be evaluated based on, for example, "RMSE/MAE (the ratio of the root mean square error to the mean absolute error)". For example, in the example of the technique disclosed herein, it is possible to evaluate a prediction model with "RMSE/MAE" of "1.253 ± 0.03" as a prediction model with high prediction accuracy.

Therefore, in the example of the technique disclosed herein, it is preferable to control the number of virtual datasets generated such that "RMSE/MAE" of the second prediction model is "1.253 ± 0.03". For example, in the example of the technique disclosed herein, it is preferable that the number of the second learning datasets used to derive the second prediction model be selected such that the ratio of the root mean square error to the mean absolute error with respect to the first learning datasets is 1.253 ± 0.03. In this way, the virtual datasets may be generated so that the prediction accuracy of the second prediction model may be further improved, and the prediction accuracy of the second prediction model may be more efficiently improved.

Details of the relationship between the number of virtual datasets generated and the prediction accuracy of the second prediction model will be described later in Example.

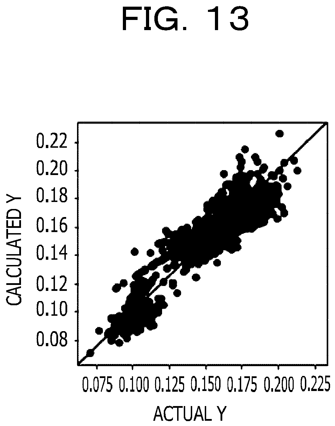

In the example of the technique disclosed herein, the first learning datasets (learning datasets from among the datasets indicating a physical property) and corresponding datasets (prediction values) corresponding to the first learning datasets in the second prediction model are compared with each other to obtain a correlation between the first learning datasets and the prediction values. The method of obtaining the predetermined correlation for the second prediction model and the like may be the same as those in the first prediction model. In another example of the technique disclosed herein for obtaining the correlation for the second prediction model, for example, the correlation between the second learning datasets and the prediction values may be obtained by comparing the second learning datasets (learning datasets used for learning of the second prediction model) with corresponding datasets (prediction values) corresponding to the second learning datasets in the second prediction model as illustrated in FIG. 13 of Example to be described later.

Subsequently, in the example of the technique disclosed herein, when the learning datasets and the prediction values for the second prediction model demonstrate the predetermined correlation, the regression coefficients of the respective candidate substances are obtained according to the second prediction model to create a prediction term. The method of creating a prediction term by obtaining the regression coefficients of the respective candidate substances in the second prediction model may be the same as that of the first prediction model.

In the example of the technique disclosed herein, as described above, it is preferable that the creation of the virtual datasets and the creation of the second prediction model be repeated until the correlation of the second prediction model with the learning datasets has the predetermined correlation. In the case where the creation of the virtual datasets and the creation of the second prediction model are repeated, for example, the prediction accuracy of the second prediction model may be efficiently improved by changing the number of virtual datasets generated.

In this case, as described above, it is preferable that the number of virtual datasets generated be changed such that the number of learning datasets used to derive the second prediction model from among the virtual datasets becomes a number with which the ratio of the root mean square error to the mean absolute error with respect to the first learning datasets approaches 1.253 ± 0.03.

<Identification of Physical Property of Mixture (Step of identifying Physical Property)>

In the example of the technique disclosed herein, physical properties of a mixture are identified by using an objective function expression including prediction terms created as described above. In the example of the technique disclosed herein, for example, the physical properties of a mixture are identified by minimizing the objective function expression including the prediction terms. For example, in the example of the technique disclosed herein, it is possible to solve a combinatorial optimization problem concerning a combination for a composition of a mixture by minimizing the objective function expression, and thereby to identify the composition of the mixture capable of optimizing the physical properties.

A method of minimizing the objective function expression used herein is not particularly limited, and may be appropriately selected in accordance with the intended purpose. A preferable method as the method of minimizing the objective function expression is to convert the objective function expression to an Ising model in the format of quadratic unconstrained binary optimization (QUBO) and minimize the value of the Ising model expression converted from the objective function expression.

As the Ising model expression converted from the objective function expression, for example, it is preferable to use a mathematical expression represented by the following expression (1). For example, in the example of the technique disclosed herein, it is preferable to identify the physical properties of the mixture based on the Ising model expression converted from the objective function expression and represented by the following expression (1).

$$E = -\sum_{i,j=0} w_{ij}\, x_i x_j - \sum_{i=0} b_i x_i \quad \cdots \text{ Expression (1)}$$

In the above expression (1), E is an objective function expression, $w_{ij}$ is a numerical value representing an interaction between an i-th bit and a j-th bit, $x_i$ is a binary variable indicating that the i-th bit is 0 or 1, and $x_j$ is a binary variable indicating that the j-th bit is 0 or 1, and $b_i$ is a numerical value representing a bias for the i-th bit.

Here, $w_{ij}$ in the above expression (1) may be obtained, for example, by extracting, for each combination of $x_i$ and $x_j$, the numerical values or the like of the respective parameters in the objective function expression before conversion to the Ising model expression, and is usually a matrix.

The first term on the right side of the above expression (1) is the sum of the products in all the combinations, without omission and duplication, of two bits selectable from all the bits, the products each obtained by multiplication of the states of two circuits and the weight value (weight).

The second term on the right side of the above expression (1) is the sum of the respective products of the bias values and the states of all the bits.

For example, it is possible to convert the objective function expression to the Ising model expression represented by the above expression (1) by extracting the parameters in the objective function expression before the conversion to the Ising model expression and obtaining $w_{ij}$ and $b_i$.

The value of the Ising model converted from the cost function as described above may be minimized within a short time by, for example, performing an annealing method using an annealing machine or the like. In the example of the technique disclosed herein, for example, it is preferable to minimize the objective function expression by the annealing method.

Examples of the annealing machine used to optimize the objective function expression include, for example, a quantum annealing machine, a semiconductor annealing machine using semiconductor technology, a machine that performs simulated annealing executed by software using a central processing unit (CPU) and a graphics processing unit (GPU), and so on. As the annealing machine, for example, Digital Annealer (registered trademark) may be used. Details of the annealing method using the annealing machine will be described later.

In the technique disclosed herein, use of the annealing method to minimize the objective function expression is not indispensable. Instead, for example, a genetic algorithm may be used to extract a combination of candidate substances (materials) that minimize the objective function expression.

<Other Steps>

The other steps are not particularly limited but may be selected according to the intended purpose as appropriate.

(Mixture Physical Property Identification Apparatus)

A mixture physical property identification apparatus disclosed herein includes: a unit that creates a prediction term for predicting at least one physical property of a mixture of a plurality of candidate substances; and a unit that identifies the physical property of the mixture by using an objective function expression including the prediction term, in which the unit that creates a prediction term includes a unit that obtains a dataset indicating the physical property of each of a plurality of mixtures each containing two or more candidate substances among the plurality of candidate substances; and a unit that sets at least some of the datasets indicating the physical property as first learning datasets, and compares the first learning datasets with corresponding datasets corresponding to the first learning datasets in a first prediction model based on the first learning datasets, when the first learning datasets and the corresponding datasets demonstrate a predetermined correlation, the prediction term is created based on regression coefficients of the respective candidate substances obtained from the first prediction model, when the first learning datasets and the corresponding datasets do not demonstrate the predetermined correlation, the unit that creates a prediction term further includes a unit that obtains virtual datasets based on an integration model obtained by integrating a plurality of prediction models generated based on the datasets indicating the physical property, and a unit that sets at least some of the virtual datasets as second learning datasets and compares the first learning datasets with corresponding datasets corresponding to the first learning datasets in a second prediction model based on the second learning datasets, and when the first learning datasets and the corresponding datasets demonstrate the predetermined correlation, the prediction term is created based on regression coefficients of the respective candidate substances obtained from the second prediction model.

The mixture physical property identification apparatus disclosed herein includes a unit that creates a prediction term and a unit that identifies the physical property, and further includes other units as requested.

The mixture physical property identification apparatus includes, for example, a memory and a processor, and further includes other units as requested. As the processor, a processor coupled to a memory so as to execute the step of creating a prediction term and the step of identifying the physical property may be preferably used.

The processor is, for example, a central processing unit (CPU), a graphics processing unit (GPU), or a combination thereof.

As described above, the mixture physical property identification apparatus disclosed herein may be, for example, an apparatus (computer) that performs the mixture physical property identification method disclosed herein. Therefore, a preferable embodiment of the mixture physical property identification apparatus disclosed herein may be similar to a preferable embodiment of the mixture physical property identification method disclosed herein.

(Mixture Physical Property Identification Program)

A mixture physical property identification program disclosed herein is a mixture physical property identification program that causes a computer to execute a process including: creating a prediction term for predicting at least one physical property of a mixture of a plurality of candidate substances; and identifying the physical property of the mixture by using an objective function expression including the prediction term, in which the creating a prediction term includes obtaining a dataset indicating the physical property of each of a plurality of mixtures each containing two or more candidate substances among the plurality of candidate substances, setting at least some of the datasets indicating the physical property as first learning datasets, and comparing the first learning datasets with corresponding datasets corresponding to the first learning datasets in a first prediction model based on the first learning datasets, when the first learning datasets and the corresponding datasets demonstrate a predetermined correlation, the prediction term is created based on regression coefficients of the respective candidate substances obtained from the first prediction model, when the first learning datasets and the corresponding datasets do not demonstrate the predetermined correlation, the creating a prediction term further includes obtaining virtual datasets based on an integration model obtained by integrating a plurality of prediction models generated based on the datasets indicating the physical property, and setting at least some of the virtual datasets as second learning datasets, and comparing the first learning datasets with corresponding datasets corresponding to the first learning datasets in a second prediction model based on the second learning datasets, when the first learning datasets and the corresponding datasets demonstrate the predetermined correlation, the prediction term is created based on regression coefficients of the respective candidate substances obtained from the second prediction model.

The mixture physical property identification program disclosed herein may be, for example, a program that causes a computer to execute the mixture physical property identification method disclosed herein. A preferable embodiment of the mixture physical property identification program disclosed herein may be similar to, for example, the preferable embodiment of the mixture physical property identification method disclosed herein.

The mixture physical property identification program disclosed herein may be created using any of various known program languages depending on conditions such as a configuration of a computer system and a type and a version of an operating system for use.

The mixture physical property identification program disclosed herein may be recorded on a recording medium such as a built-in hard disk, an external hard disk, or the like, or recorded on a recording medium such as a compact disk read-only memory (CD-ROM), a digital versatile disc read-only memory (DVD-ROM), a magneto-optical (MO) disk, or a Universal Serial Bus (USB) memory.

In a case where the mixture physical property identification program disclosed herein is recorded on the aforementioned recording medium, the mixture physical property identification program may be used directly or be used after being installed on a hard disk, as requested, via a recording medium reader included in the computer system. The mixture physical property identification program disclosed herein may be recorded in an external storage area (another computer or the like) accessible from the computer system via an information communication network. In this case, the mixture physical property identification program disclosed herein, which is recorded in the external storage area, may be used directly or be used after being installed on the hard disk, as requested, from the external storage area via the information communication network.

The mixture physical property identification program disclosed herein may be divided into certain process units, which may be recorded on multiple recording media.

(Computer Readable Recording Medium)

A computer readable recording medium disclosed herein is obtained by recording the mixture physical property identification program disclosed herein.

The computer readable recording medium disclosed herein is not particularly limited, but may be selected according to the intended purpose as appropriate. Examples thereof include a built-in hard disk, an external hard disk, a CD-ROM, a DVD-ROM, an MO disk, a USB memory, and the like.

The computer readable recording medium disclosed herein may be multiple recording media each of which records therein one of certain process units into which the mixture physical property identification program disclosed herein is divided.

Hereinafter, the example of the technique disclosed herein will be described in more detail by using configuration examples of apparatuses, flowcharts, and so on.

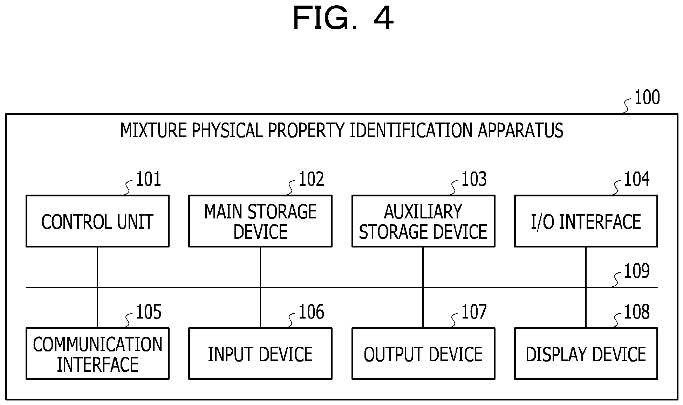

FIG. 4 illustrates a hardware configuration example of a mixture physical property identification apparatus disclosed herein.

In a mixture physical property identification apparatus 100, for example, a control unit 101, a main storage device 102, an auxiliary storage device 103, an input/output (I/O) interface 104, a communication interface 105, an input device 106, an output device 107, and a display device 108 are coupled to each other via a system bus 109.

The control unit 101 performs operations (such as four arithmetic operations, comparison operations, and annealing method operations), operation control of hardware and software, and the like. The control unit 101 may be, for example, a central processing unit (CPU), a part of an annealing machine for use in the annealing method, or a combination of them.

The control unit 101 implements various functions by, for example, executing a program (such as, for example, the mixture physical property identification program disclosed herein) read into the main storage device 102 or the like.

The processes performed by the unit that creates a prediction term (prediction term creation unit) and the unit that identifies the physical property (physical property identification unit) in the mixture physical property identification apparatus disclosed herein may be performed by, for example, the control unit 101.

The main storage device 102 stores various programs and stores data and others to be used for executing the various programs. As the main storage device 102, for example, a storage device including at least one of a read-only memory (ROM) and a random-access memory (RAM) may be used.

The ROM stores, for example, various programs such as a Basic Input/Output System (BIOS). The ROM is not particularly limited, but may be selected according to the intended purpose as appropriate, and examples thereof include a mask ROM, a programmable ROM (PROM), and the like.

The RAM functions as, for example, a work area in which the various programs stored in the ROM, the auxiliary storage device 103, and the like are expanded when executed by the control unit 101. The RAM is not particularly limited, but may be selected according to the intended purpose as appropriate, and examples thereof include a dynamic random-access memory (DRAM), a static random-access memory (SRAM), and the like.

The auxiliary storage device 103 is not particularly limited as long as it is capable of storing various kinds of information, but may be selected according to the intended purpose as appropriate. Examples thereof include a solid-state drive (SSD), a hard disk drive (HDD), and the like. The auxiliary storage device 103 may be a portable storage device such as a compact disc (CD) drive, a Digital Versatile Disc (DVD) drive, or a Blu-ray (Registered trademark) disc (BD) drive.

The mixture physical property identification program disclosed herein is stored in the auxiliary storage device 103, is loaded onto the RAM (main memory) of the main storage device 102, and is executed by the control unit 101, for example.

The I/O interface 104 is an interface for coupling to various external devices. The I/O interface 104 allows input and output of data from and to, for example, a compact disc read-only memory (CD-ROM), a Digital Versatile Disk read-only memory (DVD-ROM), a magneto-optical (MO) disk, a Universal Serial Bus (USB) memory [USB flash drive], or the like.

The communication interface 105 is not particularly limited, and any known interface may be used as appropriate. An example thereof is a wireless or wired communication device or the like.

The input device 106 is not particularly limited as long as it is capable of receiving input of various kinds of requests and information to the mixture physical property identification apparatus 100, and any known device may be used as appropriate. Examples thereof include a keyboard, a mouse, a touch panel, a microphone, and so on. When the input device 106 is a touch panel (touch display), the input device 106 may also serve as the display device 108.

The output device 107 is not particularly limited, and any known device may be used as appropriate. An example thereof is a printer or the like.

The display device 108 is not particularly limited, and any known display device may be used as appropriate. Examples thereof include a liquid crystal display, an organic EL display, and the like.

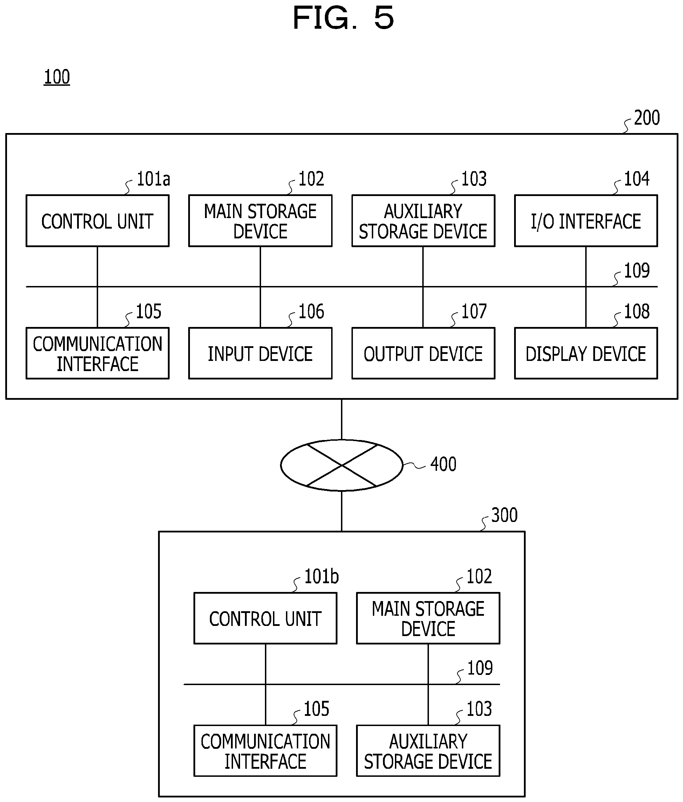

FIG. 5 illustrates another hardware configuration example of the mixture physical property identification apparatus disclosed herein.

In the example illustrated in FIG. 5, the mixture physical property identification apparatus 100 is divided into a computer 200 that performs processes such as a process of obtaining datasets on a physical property (physical property value datasets) of mixtures, a process of creating a prediction term, and a process of defining an objective function expression, and an annealing machine 300 that optimizes (minimizes) an Ising model expression. In the example illustrated in FIG. 5, the computer 200 and the annealing machine 300 in the mixture physical property identification apparatus 100 are coupled to each other via a network 400.

In the example illustrated in FIG. 5, for example, a CPU or the like may be used as a control unit 101a in the computer 200, and a device specialized for the annealing method (annealing) may be used as a control unit 101b in the annealing machine 300.

In the example illustrated in FIG. 5, for example, the computer 200 defines the objective function expression by making various kinds of settings for defining the objective function expression, and converts the defined objective function expression to the Ising model expression. The computer 200 transmits information on the values of the weight $(w_{ij})$ and the bias $(b_i)$ in the Ising model expression to the annealing machine 300 via the network 400.

The annealing machine 300 optimizes (minimizes) the Ising model expression based on the received information on the values of the weight $(w_{ij})$ and the bias $(b_i)$, and obtains the minimum value of the Ising model expression and the states of the bits that give the minimum value. The annealing machine 300 transmits the obtained minimum value of the Ising model expression and the obtained states of the bits that give the minimum value to the computer 200 via the network 400.

Subsequently, the computer 200 identifies and optimizes the physical property of the mixture based on the received states of the bits that give the minimum value to the Ising model expression.

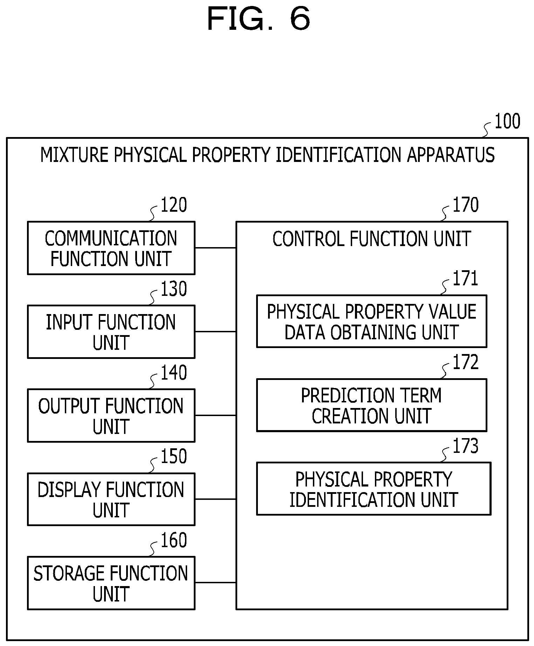

FIG. 6 illustrates a functional configuration example of the mixture physical property identification apparatus disclosed herein.

As illustrated in FIG. 6, the mixture physical property identification apparatus 100 includes a communication function unit 120, an input function unit 130, an output function unit 140, a display function unit 150, a storage function unit 160, and a control function unit 170.

The communication function unit 120 transmits and receives various kinds of data to and from an external device, for example. For example, the communication function unit 120 may receive a dataset on the physical property (performance) of each candidate substance, data on the bias and the weight in the Ising model expression converted from the objective function expression, and the like from the external device.

The input function unit 130 receives, for example, various instructions for the mixture physical property identification apparatus 100. For example, the input function unit 130 may receive input of a dataset on the physical property (performance) of each candidate substance, the data on the bias and the weight in the Ising model expression converted from the objective function expression, and the like.

The output function unit 140 prints and outputs, for example, information on the identified physical property of the mixture.

The display function unit 150 displays, for example, the information on the identified physical property of the mixture on a display.

The storage function unit 160 stores, for example, various programs, the datasets on the physical property (performance) of the respective candidate substances, the information on the identified physical property of the mixture, and the like.

The control function unit 170 includes a physical property value data obtaining unit 171, a prediction term creation unit (a unit that creates a prediction term) 172, and a physical property identification unit (a unit that identifies the physical property) 173.

The physical property value data obtaining unit 171 performs, for example, a physical property simulation (for example, a molecular dynamics simulation) for each mixture to calculate and obtain a dataset on the physical property (physical property value dataset). The prediction term creation unit 172 creates a prediction term based on the regression coefficients of the respective candidate substances by using, for example, the first prediction model or the second prediction model. The physical property identification unit 173 identifies and optimizes the physical property of the mixture by, for example, optimizing (such as minimizing) the objective function expression.

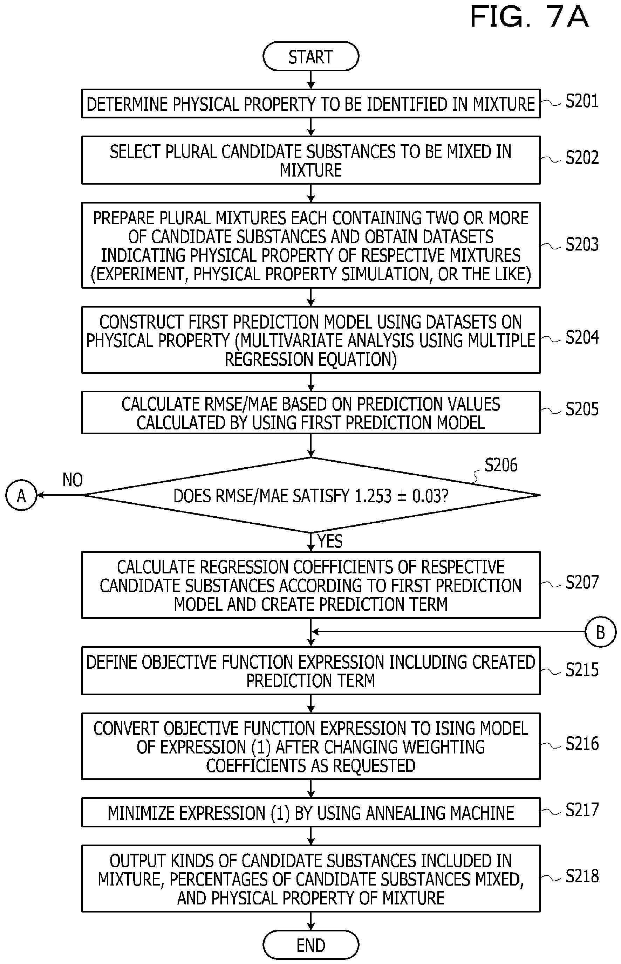

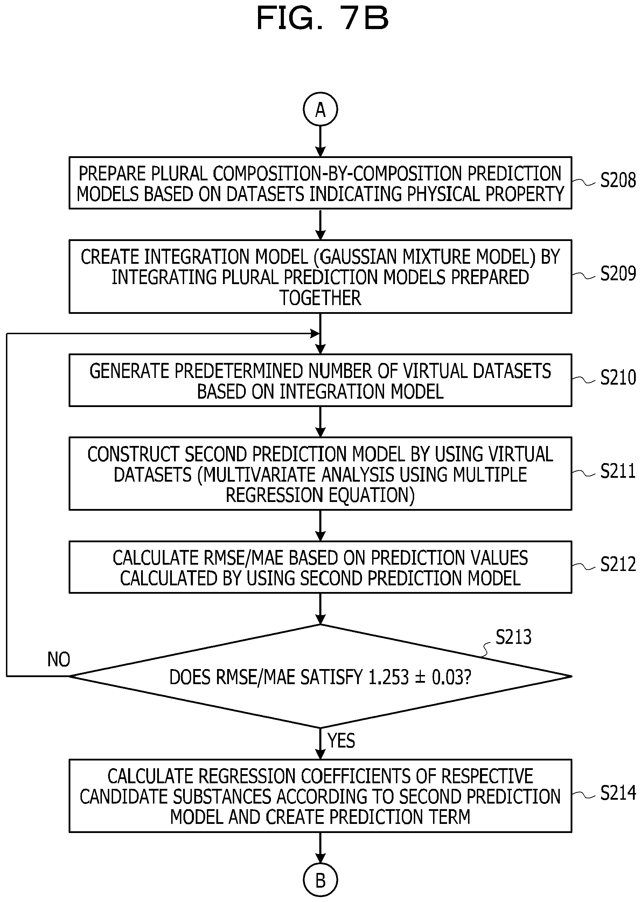

FIG. 7A and FIG. 7B illustrate an example of a flowchart of identifying and optimizing a physical property of a mixture by using the example of the technique disclosed herein.

First, the control function unit 170 determines a physical property (performance) to be identified in a mixture (S201). In S201, the control function unit 170 may determine a plurality of physical properties of the mixture as physical properties to be identified.

Next, the control function unit 170 selects a plurality of candidate substances to be mixed in the mixture (S202). For example, in S202, the control function unit 170 may extract and select a predetermined number of candidate substances by referring to, for example, a database in which information on candidate substances is recorded.

Subsequently, the physical property value data obtaining unit 171 calculates a dataset indicating the physical property (physical property value dataset) of each mixture among mixtures each containing two or more of the candidate substances (S203). For example, in S203, the physical property value data obtaining unit 171 calculates the dataset indicating the physical property (physical property value dataset) of each mixture based on results of an actual experiment and a physical property simulation for the mixtures each containing two or more of the candidate substances.

The prediction term creation unit 172 constructs a first prediction model by using the datasets indicating the physical property (S204). For example, in S204, the prediction term creation unit 172 sets some of the datasets indicating the physical property as test datasets and the rest as first learning datasets, and constructs the first prediction model by performing a multivariate analysis using a multiple regression equation based on the first learning datasets.

Next, the prediction term creation unit 172 calculates RMSE/MAE (the ratio of the root mean square error to the mean absolute error) based on the prediction values calculated by using the first prediction model (S205). For example, in S205, the prediction term creation unit 172 calculates RMSE/MAE in the prediction values of the physical property predicted by using the first prediction model and the first learning datasets corresponding to the prediction values.

Subsequently, the prediction term creation unit 172 determines whether or not RMSE/MAE satisfies 1.253 $\pm$ 0.03 (S206). In S206, the prediction term creation unit 172 advances the process to S207 when it is determined that RMSE/MAE satisfies 1.253 $\pm$ 0.03, or advances the process to S208 when it is determined that RMSE/MAE does not satisfy 1.253 $\pm$ 0.03.

When it is determined that RMSE/MAE satisfies 1.253 $\pm$ 0.03, the prediction term creation unit 172 obtains the regression coefficients of the respective candidate substances according to the first prediction model to create the prediction term (S207).

On the other hand, when it is determined that RMSE/MAE does not satisfy 1.253 $\pm$ 0.03, the prediction term creation unit 172 prepares a plurality of composition-by-composition prediction models based on the datasets indicating the physical property (S208). For example, in S208, the prediction term creation unit 172 creates and prepares, using the datasets indicating the physical property for each kind of combinations of candidate substances, a prediction model capable of predicting the physical property value that the combination may take along with a change in the component ratio (mixture ratio).

Next, the prediction term creation unit 172 creates an integration model (for example, a Gaussian mixture model) obtained by integrating the plurality of prediction models thus prepared (S209).

Subsequently, the prediction term creation unit 172 generates a predetermined number of virtual datasets based on the integration model (S210). For example, in S210, the prediction term creation unit 172 generates the virtual datasets according to the integration model by generating datasets in which physical property values are randomly set so as to satisfy a probability distribution in the integration model, for example.

The prediction term creation unit 172 constructs a second prediction model by using the virtual datasets (S211). For example, in S211, the prediction term creation unit 172 constructs the second prediction model by using some of the virtual datasets generated based on the integration model as second learning datasets and performing a multivariate analysis using a multiple regression equation based on the second learning datasets.

Next, the prediction term creation unit 172 calculates RMSE/MAE (the ratio of the root mean square error to the mean absolute error) based on the prediction values calculated by using the second prediction model (S212).

For example, in S212, the prediction term creation unit 172 calculates RMSE/MAE in the prediction values of the physical property predicted by using the second prediction model and the first learning datasets corresponding to the prediction values.

Subsequently, the prediction term creation unit 172 determines whether or not RMSE/MAE satisfies 1.253 $\pm$ 0.03 (S213). In S213, the prediction term creation unit 172 advances the process to S214 when it is determined that RMSE/MAE satisfies 1.253 $\pm$ 0.03, or returns the process to S210 when it is determined that RMSE/MAE does not satisfy 1.253 $\pm$ 0.03.

When the process is returned to the S210 because it is determined that RMSE/MAE does not satisfy 1.253 $\pm$ 0.03, the number of virtual datasets generated in S210 (number of datasets generated) is changed.

When it is determined that RMSE/MAE satisfies 1.253 $\pm$ 0.03, the prediction term creation unit 172 obtains the regression coefficients of the respective candidate substances according to the second prediction model, and creates a prediction term (S214).

The physical property identification unit 173 defines an objective function expression including the prediction term created in S207 or S214 (S215). In this step, the physical property identification unit 173 causes the objective function expression to contain the above-described prediction term and also contain weighting coefficients for respective parameters and a constraint term on a search for a composition of a mixture.

Next, the physical property identification unit 173 changes the weighting coefficients as requested, and then converts the objective function expression to the Ising model represented by the following expression (1) (S216). For example, in S216, the physical property identification unit 173 extracts the parameters in the defined objective function expression, and obtains $b_i$ (bias) and $w_{ij}$ (weight) in the following expression (1), thereby converting the objective function expression to the Ising model expression represented by the following expression (1).

$$E = - \sum_{i,j=0} w_{ij} x_i x_j - \sum_{i=0} b_i x_i$$

Expression (1)

In the above expression (1), E is an objective function expression, $w_{ij}$ is a numerical value representing an interaction between an i-th bit and a j-th bit, $x_i$ is a binary variable indicating that the i-th bit is 0 or 1, and $x_j$ is a binary variable indicating that the j-th bit is 0 or 1, and $b_i$ is a numerical value representing a bias for the i-th bit.

Next, the physical property identification unit 173 minimizes the above expression (1) by using an annealing machine (S217). For example, in S217, the physical property identification unit 173 executes the ground-state search on the above expression (1) by using the annealing method to calculate the lowest energy of the above expression (1), thereby searching for the composition of the mixture that may minimize the objective function expression.

Then, the physical property identification unit 173 outputs, based on the result of minimizing the above expression (1), the kinds of candidate substances included in the mixture, the percentages of the candidate substances mixed (the composition of the mixture), and the physical property (physical property value) of the mixture under the condition that the objective function expression takes the minimum value (S218). After outputting the composition and the physical property of the mixture, the physical property identification unit 173 ends the process.

Although the sequence of identifying the physical property of a mixture by using the example of the technique disclosed herein has been described in accordance with a specific order in FIG. 7A and FIG. 7B, the order of steps in the technique disclosed herein may be changed as appropriate within a technically possible range. In the technique disclosed herein, some of the steps may be collectively performed within a technically possible range.

An example of an annealing method and an annealing machine will be described below.

The annealing method is a method of obtaining a solution stochastically by using a random number value or a superposition of quantum bits. Hereinafter, a problem of minimizing a value of an evaluation function desired to be optimized will be described as an example, and the value of the evaluation function will be referred to as energy. When the value of the evaluation function is desired to be maximized, a sign of the evaluation function may be changed.

First, starting from initial states where one discrete value is assigned to each of variables, a state transition from current states (a combination of the values of the variables) to selected states close to the current states (for example, the states where only one of the variables is changed) is considered. A change in energy associated with the state transition is calculated, and whether to accept the state transition and change the states or to maintain the original states without accepting the state transition is stochastically determined according to the calculated value of the change in energy. When an acceptance probability for a case where the energy decreases is selected to be higher than the acceptance probability for a case where the energy increases, it is expected that a state change occurs in a direction in which the energy decreases on average and the states transition to more appropriate states over time. Thus, there is a possibility of finally obtaining an approximate solution giving energy at an optimal solution or close to an optimal value.

If the state transition is deterministically accepted in a case where the energy decreases or rejected in a case here the energy increases, the change in energy will be weakly decreasing over time. However, once a local solution is reached, the change will not occur any more. Since an extraordinarily large number of local solutions exist in a discrete optimization problem as described above, the states are often stuck at a local solution that is not very close to the optimal value. For this reason, in solving a discrete optimization problem, it is important to stochastically determine whether or not to accept the states.

In the annealing method, it has been proved that the states reach the optimal solution in the limit of an infinite number of times (number of iterations) by determining the acceptance probability of the state transition as follows.

Hereinafter, a sequence of a method of determining an optimal solution using the annealing method will be described.

(1) For an energy change (energy decrease) value ($-\Delta E$) associated with a state transition, the acceptance probability p for the state transition is determined by any of the following functions f().

$$p(\Delta E, T) = f(-\Delta E / T)$$

(Expression 1-1)

$$f_{metro}(x) = \min(1, e^x)$$

(Metropolis method)(Expression 1-2)

$$f_{Gibbs}(x) = \frac{1}{1+e^{-x}} \qquad \text{(Gibbs method)(Expression 1-3)}$$

Here, T is a parameter called a temperature value and may be changed, for example, as follows.

(2) The temperature value T is logarithmically decreased according to the number of iterations t as represented by the following expression.

$$T = \frac{T_0 \log(c)}{\log(t+c)} \qquad \text{(Expression 2)}$$

Here, $T_0$ denotes an initial temperature value and is desirably set to a sufficiently large value depending on the problem.

In a case where the acceptance probability expressed by the expression (1) is used and the steady states are reached after sufficient iterations, the probability of each state being occupied follows the Boltzmann distribution in a thermal equilibrium state in thermodynamics.

When the temperature gradually decreases from a high temperature, the probability of a low energy state being occupied increases. For this reason, when the temperature decreases sufficiently, it is expected to obtain the low energy states. This method is referred to as an annealing method (or simulated annealing method) because this behavior resembles a state change in annealing of a material. The stochastic occurrence of a state transition where the energy increases is equivalent to thermal excitation in physics.

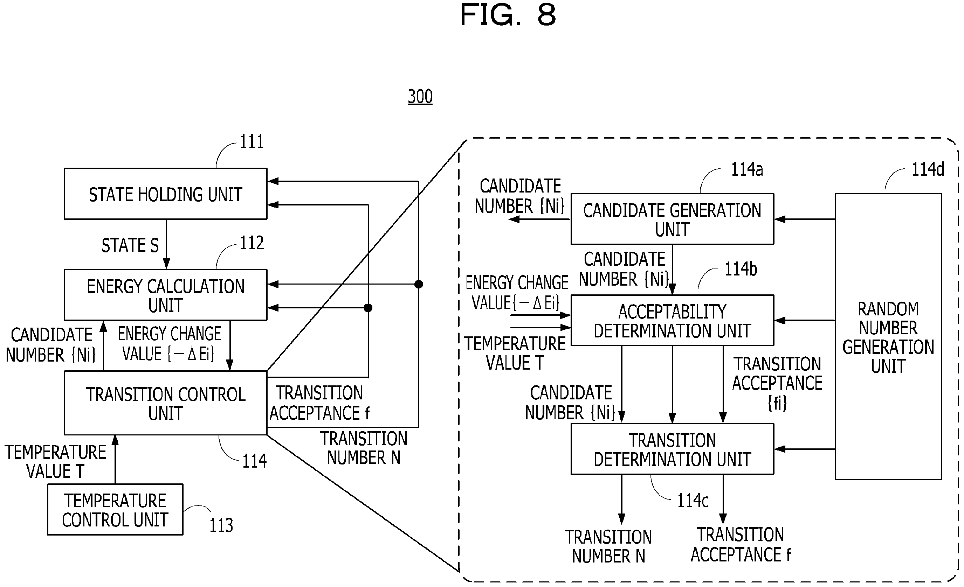

FIG. 8 illustrates an example of a functional configuration of an annealing machine that performs the annealing method. Although the following description will also explain a case where multiple candidates for the state transition are generated, one transition candidate is generated at one time in the basic annealing method.

An annealing machine 300 includes a state holding unit 111 that holds current states S (values of multiple state variables). The annealing machine 300 also includes an energy calculation unit 112 that calculates an energy change value {-ΔEi} for each of state transitions in a case where the state transition occurs from the current states S as a result of changing any of the values of the multiple state variables. The annealing machine 300 includes a temperature control unit 113 that controls a temperature value T and a transition control unit 114 that controls a state change. The annealing machine 300 may be configured as a part of the mixture physical property identification apparatus 100 described above.

The transition control unit 114 stochastically determines whether or not to accept any one of multiple state transitions, depending on a relative relationship between the energy change value {-ΔEi} and thermal excitation energy based on the temperature value T, the energy change value {-ΔEi}, and a random number value.

The transition control unit 114 includes a candidate generation unit 114a that generates candidates for a state transition, and an acceptability determination unit 114b that stochastically determines whether or not the state transition in each of the candidates is acceptable based on the energy change value {-ΔEi} and the temperature value T. The transition control unit 114 includes a transition determination unit 114c that determines a candidate to be actually employed from the candidates determined as acceptable, and a random number generation unit 114d that generates a probability variable.

An operation in one iteration by the annealing machine 300 is as follows.

First, the candidate generation unit 114a generates one or more candidates (candidate No. {Ni}) for a state transition to the next states from the current states S held by the state holding unit 111. The energy calculation unit 112 calculates an energy change value {-ΔEi} for the state transition specified in each of the candidates by using the current states S and the candidate for the state transition. The acceptability determination unit 114b determines each of the state transitions as acceptable with the acceptance probability expressed by the above expression (1) according to the energy change value {-ΔEi} for the state transition by using the temperature value T generated in the temperature control unit 113 and the probability variable (random number value) generated in the random number generation unit 114d.

The acceptability determination unit 114b outputs the acceptability {fi} of each of the state transitions. In a case where multiple state transitions are determined as acceptable, the transition determination unit 114c randomly selects one of them by using a random number value. The transition determination unit 114c then outputs the transition number N of the selected state transition, and the transition acceptability f. In a case where there is a state transition accepted, the values of the state variables stored in the state holding unit 111 are updated according to the accepted state transition.

Starting with the initial states, the above-described operation is iterated while causing the temperature control unit 113 to decrease the temperature value, and is ended when satisfying an end determination condition such as a condition where a certain number of iterations is reached or the energy falls below a predetermined value. The answer output by the annealing machine 300 is the states at the end.

The annealing machine 300 illustrated in FIG. 8 may be implemented by using, for example, a semiconductor integrated circuit. For example, the transition control unit 114 may include a random number generation circuit that functions as the random number generation unit 114d, a comparator circuit that functions as at least a part of the acceptability determination unit 114b, a noise table to be described later, and so on.

Regarding the transition control unit 114 illustrated in FIG. 8, a mechanism to accept a state transition with the acceptance probability expressed by the expression (1) will be described in more detail.

A circuit that outputs 1 with an acceptance probability p and outputs 0 with an acceptance probability (1-p) may be implemented by using a comparator that has two inputs A and B, and that outputs 1 when A > B and outputs 0 when A < B and by inputting the acceptance probability p to the input A and inputting a uniform random number having a value in the unit interval [0, 1) to the input B. Thus, it is possible to achieve the above function when the value of the acceptance probability p calculated by using the expression (1) based on the energy change value and the temperature value T is input to the input A of the comparator.

For example, provided that f denotes a function used in the expression (1), and u denotes a uniform random number having a value in the unit interval [0, 1), a circuit that outputs 1 when $f(\Delta E/T)$ is greater than u achieves the above function.

The circuit may achieve the same function as described above even when modified as follows.

Even when the same monotonically increasing function is applied to two numbers, the two numbers maintain the same magnitude relationship. Therefore, even when the same monotonically increasing function is applied to the two inputs of the comparator, the same output is obtained. When an inverse function $f^{-1}$ of f is used as this monotonically increasing function, it is seen that the circuit may be modified to a circuit that outputs 1 when $-\Delta E/T$ is greater than $f^{-1}(u)$. Since the temperature value T is positive, it is seen that the circuit may be one that outputs 1 when $-\Delta E$ is greater than $Tf^{-1}(u)$.

The transition control unit 114 in FIG. 8 may include a noise table which is a conversion table for realizing the inverse function $f^{-1}(u)$, and which outputs a value of any of the following functions for an input of each discrete value within the unit interval [0, 1).

$$f_{metro}^{-1}(u) = \log(u) \qquad \text{(Expression 3-1)}$$

$$f_{Gibbs}^{-1}(u) = \log\left(\frac{u}{1-u}\right) \qquad \text{(Expression 3-2)}$$

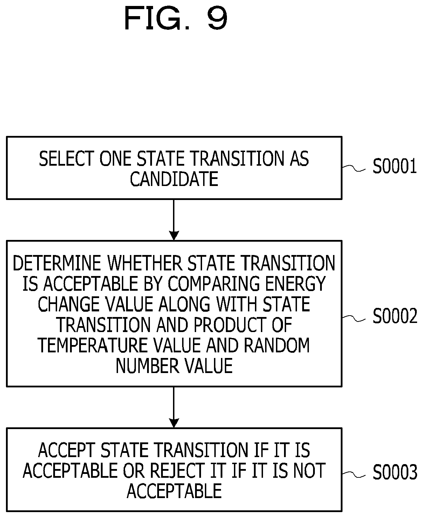

FIG. 9 illustrates one example of an operation flow of the transition control unit 114. The operation flow illustrated in FIG. 9 includes a step of selecting one state transition as a candidate (S0001), a step of determining whether the state transition is acceptable or not by comparing the energy change value for the state transition with a product of a temperature value and a random number value (S0002), and a step of accepting the state transition when the state transition is acceptable or rejecting the state transition when the state transition is not acceptable (S0003).

[Example]

Although Example of the technique disclosed herein will be described, the technique disclosed herein is not limited to this Example at all.

As Example, a prediction term for predicting a physical property of a mixture was created by using an example of the mixture physical property identification apparatus disclosed herein, and the relationship between the number of virtual datasets generated and the prediction accuracy of the second prediction model was examined. In Example, assuming a mixed refrigerant as an example of a mixture, the prediction term for predicting the physical property of the mixture was created in accordance with the sequence of S201 to S214 illustrated in the flowchart of FIG. 7A and FIG. 7B, by using an optimization apparatus having a hardware configuration as illustrated in FIG. 5 and a functional configuration as illustrated in FIG. 6.

In Example, the following five kinds of candidate substances are used as candidate substances (materials) to be explanatory variables in the prediction model. A hydrofluoroolefin (HFO) refrigerant, "Opteon SF-10 (methoxyperfluoroheptene, $C_7F_{13}OCH_3$)"; n-Pentane; Methyl alcohol; Diethylene glycol monobutyl ether (DGME); Diethyl ether.

In Example, 40 mixtures were each prepared by arbitrarily selecting three candidate substances from the above five candidate substances (explanatory variables) and a composition ratio thereof, and the thermal conductivity of each of these mixtures was calculated. The molecular dynamics calculation program "LAMMPS" was used for this calculation (simulation) of the thermal conductivity of each mixture of three components.

In Example, the thermal conductivity of the mixture of three components was calculated according to the following procedure.

First, energy equilibration of mixed molecules arranged in a cubic cell was performed. In this equilibration, as a calculation system, created was a structure in which the candidate substances were distributed at a predetermined molar ratio such that the mixture of the three components includes 60 molecules (a structure in which the molecules to be mixed are arranged in the cell).

The calculation of the equilibration of the molecular structure in LAMMPS was performed under the conditions of a temperature of 298.2 K (25°C), a pressure of 1 atm, and a simulation time step of 0.5 fsec (0.5 femto seconds).

After the equilibration of the mixed molecules, non-equilibrium molecular dynamics (MDs) simulation was performed, and the thermal conductivity was calculated by using the Muller-Plathe method. In the non-equilibrium molecular dynamics simulation, a high temperature region and a low temperature region were provided in the calculation system, and the thermal conductivity was analyzed by using Fourier's law based on a heat flux and a temperature gradient generated between the high and low temperature regions.

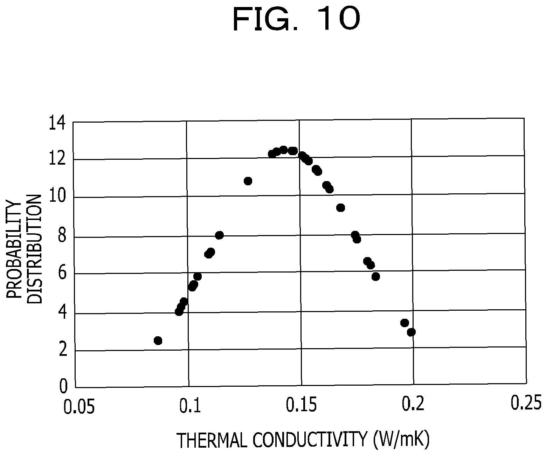

FIG. 10 illustrates an example of a distribution of the thermal conductivity of the 40 mixtures obtained by the non-equilibrium molecular dynamics simulation described above. As illustrated in FIG. 10, the distribution of the thermal conductivity of the 40 mixtures is a normal distribution, and it was confirmed that there was no large bias in the distribution of the thermal conductivity of the 40 mixtures in each of which the three components were arbitrarily selected and combined.

Next, in Example, the 40 thermal conductivity datasets (datasets indicating the physical property) were randomly divided into 32 datasets and 8 datasets such that learning datasets containing 80% of the thermal conductivity datasets and test datasets containing 20% thereof were created, respectively. In Example, a prediction model (first prediction model) of the thermal conductivity was constructed by performing a regression analysis on the learning datasets (first learning datasets). For example, in Example, the prediction model was constructed by performing the least squares regression. The least squares regression was performed by using "Scikit-learn" which is a machine learning library of Python 3.

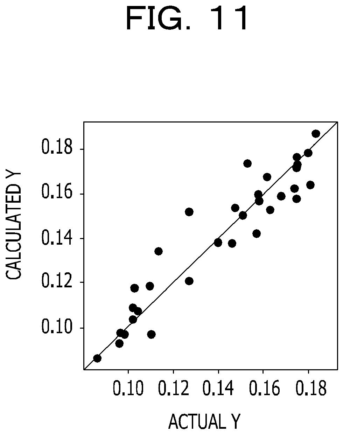

Subsequently, in Example, the prediction values were calculated by using the constructed prediction model. FIG. 11 illustrates a relationship between the prediction values calculated from the prediction model constructed by using the 32 learning datasets and the actual values (learning datasets). In FIG. 11, the vertical axis (Calculated Y) indicates the prediction value calculated from the prediction model, the horizontal axis (Actual Y) indicates the actual value (learning dataset), and the diagonal straight line indicates the prediction model (regression line).

Based on the data illustrated in FIG. 11, "RMSE/MAE (the ratio of the root mean square error to the mean absolute error)" was calculated to be "1.360".

Thus, since "RMSE/MAE" did not satisfy "1.253 ± 0.03", the accuracy of the constructed prediction model (first prediction model) of the thermal conductivity was not sufficient (the predetermined correlation was not demonstrated). For this reason, the second prediction model (Gaussian mixture model) was constructed.

For example, virtual datasets were generated by assuming the Gaussian mixture model and using the 40 thermal conductivity datasets of the mixtures obtained by the non-equilibrium molecular dynamics simulation.

A relationship between the number of virtual datasets generated and a thermal conductivity prediction model (second prediction model) constructed based on the virtual datasets was examined. In this examination, a thermal conductivity prediction model (second prediction model) was constructed for each of the cases where the number of virtual datasets generated was set to 200, 500, 1000, 2000, 5000, 10000, and 20000, and the prediction models were evaluated.

As indexes for evaluation of the prediction models, the coefficient of determination ($r^2$), the root mean square error (RMSE), the mean absolute error (MAE), and RMSE/MAE were calculated, and which of the indexes has an ability to reflect the accuracy (feature) of the prediction model was examined.

In the calculation of these indices, 80% of the virtual datasets generated according to the Gaussian mixture model were used as learning datasets for the second prediction model (for training, second learning datasets). The calculation results of the indexes are presented in Table 1.

Table 1

| NUMBER OF DATASETS | | 40 | 200 | 500 | 1000 | 2000 | 5000 | 10000 | 20000 | |
|---|---|---|---|---|---|---|---|---|---|---|
| FOR TRAINING (CONSTRUCTION OF PERFORMANCE PREDICTION MODEL) | | 32 | 160 | 400 | 800 | 1600 | 4000 | 8000 | 16000 | |
| FOR TEST (MODEL VERIFICATION) | | | 8 | 40 | 100 | 200 | 400 | 1000 | 2000 | 4000 |
| PREDICTION MODEL | r2 | | 0.8930314 | 0.89991 | 0.88 6902 | 0.893824 | 0.891507 | 0.890291 | 0.889269 | 0.889198 |
| | RMSE | | 0.0102074 | 0.009946 | 0.010728 | 0.010276 | 0.010435 | 0.010464 | 0.010573 | 0.010614 |
| | MAE | | 0.0075087 | 0.007581 | 0.0082 | 0.001976 | 0.008321 | 0.008148 | 0.008137 | 0.00791 |
| | RMSE/MAE | | 1.3594148 | 1.311921 | 13.08293 | 1.28834 | 1.254134 | 1.284275 | 1.299371 | 1.34187 |

As seen from Table 1, the values of $r^2$, RMSE, and MAE do not significantly change even when the number of virtual datasets generated is increased.

On the other hand, the value of RMSE/MAE significantly changes depending on the number of virtual datasets generated. When the number of virtual datasets generated is "2000", RMSE/MAE takes a value around "1.253", and makes it possible to determine that the accuracy of the prediction model (second prediction model) is high.

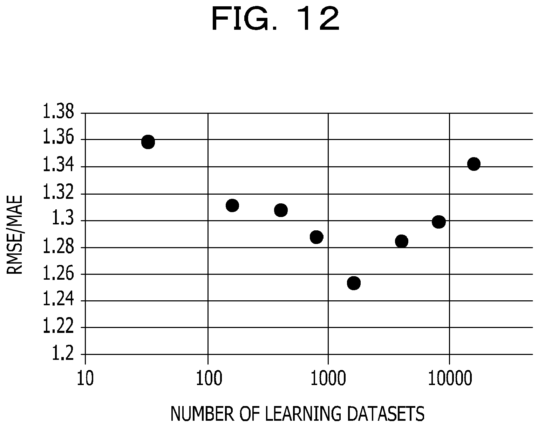

For example, FIG. 12 illustrates the relationship between the number of virtual datasets generated and RMSE/MAE in the thermal conductivity prediction model (second prediction models) constructed by using 80% of generated virtual datasets as the learning datasets. As illustrated in FIG. 12, RMSE/MAE is close to "1.253" when 2000 datasets are generated from the 40 thermal conductivity datasets. Thus, it is possible to consider that the prediction accuracy of the prediction model in the case where the number of virtual datasets generated is 2000 is particularly high.

With reference to FIG. 12, RMSE/MAE in the prediction model with high prediction accuracy in the case where the number of virtual datasets generated is 2000 and RMSE/MAE in any of the other prediction models have a difference larger than "0.03". This means that the prediction model with RMSE/MAE satisfying "$1.253 \pm 0.03$" has particularly high prediction accuracy as compared with the other prediction models.

As a result of the above examination, it is seen that it is preferable to use RMSE/MAE for evaluating the prediction accuracy of the prediction model. As described above, regarding RMSE/MAE, it is possible to evaluate that the accuracy of the prediction model is high when RMSE/MAE takes a value around "1.253" as seen from the above equation (17).

FIG. 13 illustrates a relationship between prediction values calculated by using a prediction model constructed by using 1600 virtual datasets from among 2000 virtual datasets as learning datasets and actual values (second learning datasets) corresponding to the prediction values. In FIG. 13, the vertical axis (Calculated Y) indicates the prediction value calculated from the prediction model, the horizontal axis (Actual Y) indicates the actual value (learning dataset), and the diagonal straight line indicates the prediction model (regression line).

As illustrated in FIG. 13, in the prediction model constructed by using the 1600 virtual datasets from among the 2000 virtual datasets as the learning datasets, it is seen that the datasets are concentrated around the prediction model, which means that the prediction accuracy of the prediction model is high.

In Example, the regression coefficients (partial regression coefficients) of the respective candidate substances were obtained from the prediction model constructed by using the 1600 virtual datasets from among the 2000 virtual datasets as the learning datasets.

For example, in Example, the regression coefficient of each of the candidate substances was obtained by outputting the standard regression coefficient in the constructed prediction model by using a function for regression analysis in the "Scikit-learn" library. The result is illustrated in Table 2.

Table 2

| EXPLANATORY VARIABLE | REGRESSION COEFFICIENT |
|---|---|
| SF-10 | 0.004835 |
| n-PENTANE | 0.005720 |
| METHANOL | 0.005942 |
| DGME | 0.006403 |
| DIETHYL ETHER | 0.005837 |
| CONSTANT TERM | -0.430103 |

As a prediction term for the thermal conductivity, a term is created in which products, each being a product of one of the regression coefficients presented in Table 2 and the component percentage of the corresponding candidate substance (material), and a value of a constant term are added up. Thus, it is possible to predict and identify the thermal conductivity of a mixture prepared by each combination from among the five candidate substances.

As described above, in Example, the first prediction model and the second prediction model for the thermal conductivity, which is an example of the physical properties of the mixture, were constructed, and thereby the prediction term capable of predicting the thermal conductivity with higher accuracy was successfully created.

In the example of the technique disclosed herein, a physical property of a mixture is identified using an objective function expression including a prediction term created in this manner. Thus, it is possible to predict and identify a physical property of any mixture with high accuracy even in a case of predicting the physical property for which a mathematical expression capable of estimating the physical property in a mixed state (physical property estimating equation) does not exist.

The following appendices are further disclosed regarding the above embodiments.

**Claims**

1. A mixture physical property identification method for a computer to execute a process comprising:

   creating a prediction term for predicting at least one physical property of a mixture of a plurality of candidate substances; and
   identifying the physical property of the mixture by using an objective function expression including the prediction term, wherein
   the creating includes
   obtaining a dataset indicating the physical property of each of a plurality of mixtures each containing two or more candidate substances among the plurality of candidate substances,
   setting at least some of the datasets indicating the physical property as first learning datasets, and
   comparing the first learning datasets with corresponding datasets corresponding to the first learning datasets in a first prediction model based on the first learning datasets,
   when the first learning datasets and the corresponding datasets demonstrate a certain correlation, the prediction term is created based on regression coefficients of the respective candidate substances obtained from the first prediction model,
   when the first learning datasets and the corresponding datasets do not demonstrate the certain correlation, the creating further includes
   obtaining virtual datasets based on an integration model obtained by integrating a plurality of prediction models generated based on the datasets indicating the physical property, and
   setting at least some of the virtual datasets as second learning datasets, and comparing the first learning datasets with corresponding datasets corresponding to the first learning datasets in a second prediction model based on the second learning datasets,
   when the first learning datasets and the corresponding datasets demonstrate the certain correlation, the prediction term is created based on regression coefficients of the respective candidate substances obtained from the second prediction model.

2. The mixture physical property identification method according to claim 1, wherein

   the objective function expression is represented by the following expression:

   $$E = \alpha \cdot [\text{Mixture Physical Property Prediction 1}] + \beta \cdot [\text{Mixture Physical Property Prediction 2}] + \gamma \cdot [\text{Mixture Physical Property Prediction 3}] + \ldots + \text{Constraint Term,}$$

   where E is the objective function expression, and $\alpha$, $\beta$, and $\gamma$ are weighting coefficients.

3. The mixture physical property identification method according to claim 1 or 2, wherein
   the certain correlation is defined such that a ratio of a root mean square error to a mean absolute error with respect to at least either of the first learning datasets or the second learning datasets is 1.253 ± 0.03.

4. The mixture physical property identification method according to any one of claims 1 to 3, wherein
   at least one of the first prediction model and the second prediction model is derived by a multiple regression equation based on the first learning datasets or the second learning datasets.

5. The mixture physical property identification method according to any one of claims 1 to 4, wherein
   the number of second learning datasets to be used for deriving the second prediction model is selected such that a ratio of a root mean square error to a mean absolute error with respect to the first learning datasets is 1.253 ± 0.03.

6. The mixture physical property identification method according to any one of claims 1 to 5, wherein
   the physical property of the mixture is identified by minimizing a value of the objective function expression.

7. The mixture physical property identification method according to claim 6, wherein

the identifying the physical property includes identifying the physical property of the mixture based on the objective function expression converted to an Ising model represented by the following expression (1):

$$E = -\sum_{i,j=0} w_{ij}\, x_i x_j \;-\; \sum_{i=0} b_i x_i \qquad \text{Expression (1)}$$

in the expression (1),
E is the objective function expression,
$W_{ij}$ is a numerical value representing an interaction between an i-th bit and a j-th bit,
$b_i$ is a numerical value representing a bias for the i-th bit,
$x_i$ is a binary variable indicating that the i-th bit is 0 or 1, and
$x_j$ is a binary variable indicating that the j-th bit is 0 or 1.

8. The mixture physical property identification method according to claim 6 or 7, wherein
the identifying the physical property includes minimizing the objective function expression by an annealing method.

9. A mixture physical property identification apparatus comprising:

a creating unit that creates a prediction term for predicting at least one physical property of a mixture of a plurality of candidate substances; and
an identifying unit that identifies the physical property of the mixture by using an objective function expression including the prediction term, wherein
the creating unit includes
a first obtaining unit that obtains a dataset indicating the physical property of each of a plurality of mixtures each containing two or more candidate substances among the plurality of candidate substances; and
a first setting unit that sets at least some of the datasets indicating the physical property as first learning datasets, and compares the first learning datasets with corresponding datasets corresponding to the first learning datasets in a first prediction model based on the first learning datasets,
when the first learning datasets and the corresponding datasets demonstrate a certain correlation, the prediction term is created based on regression coefficients of the respective candidate substances obtained from the first prediction model,
when the first learning datasets and the corresponding datasets do not demonstrate the certain correlation, the creating unit further includes
a second obtaining unit that obtains virtual datasets based on an integration model obtained by integrating a plurality of prediction models generated based on the datasets indicating the physical property, and
a second setting unit that sets at least some of the virtual datasets as second learning datasets and compares the first learning datasets with corresponding datasets corresponding to the first learning datasets in a second prediction model based on the second learning datasets, and
when the first learning datasets and the corresponding datasets demonstrate the certain correlation, the prediction term is created based on regression coefficients of the respective candidate substances obtained from the second prediction model.

10. A mixture physical property identification program that causes at least one computer to execute a process, the process comprising:

creating a prediction term for predicting at least one physical property of a mixture of a plurality of candidate substances; and
identifying the physical property of the mixture by using an objective function expression including the prediction term, wherein
the creating includes
obtaining a dataset indicating the physical property of each of a plurality of mixtures each containing two or more candidate substances among the plurality of candidate substances,
setting at least some of the datasets indicating the physical property as first learning datasets, and

comparing the first learning datasets with corresponding datasets corresponding to the first learning datasets in a first prediction model based on the first learning datasets,

when the first learning datasets and the corresponding datasets demonstrate a certain correlation, the prediction term is created based on regression coefficients of the respective candidate substances obtained from the first prediction model,

when the first learning datasets and the corresponding datasets do not demonstrate the certain correlation, the creating further includes

obtaining virtual datasets based on an integration model obtained by integrating a plurality of prediction models generated based on the datasets indicating the physical property, and

setting at least some of the virtual datasets as second learning datasets, and comparing the first learning datasets with corresponding datasets corresponding to the first learning datasets in a second prediction model based on the second learning datasets,

when the first learning datasets and the corresponding datasets demonstrate the certain correlation, the prediction term is created based on regression coefficients of the respective candidate substances obtained from the second prediction model.

# FIG. 1

MATERIAL 1   MATERIAL 2   MATERIAL 3   MATERIAL 4   ...   MATERIAL N

PHYSICAL PROPERTIES
(PERFORMANCE)

30%   20%   50%

MIXTURE

PHYSICAL PROPERTY 1,
PHYSICAL PROPERTY 2,
PHYSICAL PROPERTY 3, ...

BOILING POINT
MELTING POINT
DENSITY
THERMAL CONDUCTIVITY
PRESSURE
SPECIFIC HEAT
VISCOSITY
CONDUCTIVITY
...

EP 4 002 376 A1

# FIG. 2

START

S101

DETERMINE PHYSICAL PROPERTY (PERFORMANCE) TO BE
IDENTIFIED IN MIXTURE

S102

SELECT PLURAL CANDIDATE SUBSTANCES TO BE MIXED IN
MIXTURE

S103

COLLECT AND LIST PHYSICAL PROPERTY VALUES OF
CANDIDATE SUBSTANCES FROM DB OR THE LIKE

S104

ESTIMATE PHYSICAL PROPERTY OF MIXTURE FROM PHYSICAL
PROPERTY OF EACH CANDIDATE SUBSTANCE BY USING
PHYSICAL PROPERTY ESTIMATING EQUATION

S105

DEFINE OBJECTIVE FUNCTION EXPRESSION WITH PHYSICAL
PROPERTY OF MIXTURE AS PARAMETER

S106

OPTIMIZE OBJECTIVE FUNCTION EXPRESSION

S107

OUTPUT KINDS OF CANDIDATE SUBSTANCES INCLUDED IN
MIXTURE, PERCENTAGES OF CANDIDATE SUBSTANCES MIXED,
AND PERFORMANCE OF MIXTURE

END

## FIG. 3A

DISTRIBUTION OF MIXTURE OF
A, B, AND C (EXPLANATORY
VARIABLES A, B, AND C)

DISTRIBUTION OF MIXTURE OF
C, D, AND E (EXPLANATORY
VARIABLES C, D, AND E)

PROBABILITY
DISTRIBUTION

PHYSICAL
PROPERTY VALUE

## FIG. 3B

MIXTURE OF A, B,
AND C

PHYSICAL
PROPERTY VALUE

PERCENTAGE OF B

PERCENTAGE OF A

# FIG. 3C

MIXTURE OF C, D, AND E

PHYSICAL PROPERTY VALUE

PERCENTAGE OF D

PERCENTAGE OF C

# FIG. 3D

GAUSSIAN MIXTURE MODEL

PROBABILITY DISTRIBUTION

PHYSICAL PROPERTY VALUE

# FIG. 4

100

## MIXTURE PHYSICAL PROPERTY IDENTIFICATION APPARATUS

| 101 | 102 | 103 | 104 |
|---|---|---|---|
| CONTROL UNIT | MAIN STORAGE DEVICE | AUXILIARY STORAGE DEVICE | I/O INTERFACE |

109

| 105 | 106 | 107 | 108 |
|---|---|---|---|
| COMMUNICATION INTERFACE | INPUT DEVICE | OUTPUT DEVICE | DISPLAY DEVICE |

# FIG. 5

<u>100</u>

┌─ 200

┌─ 101a        ┌─ 102           ┌─ 103            ┌─ 104
CONTROL UNIT   MAIN STORAGE     AUXILIARY        I/O INTERFACE
               DEVICE           STORAGE DEVICE

                                                  ┌─ 109

┌─ 105         ┌─ 106           ┌─ 107            ┌─ 108
COMMUNICATION  INPUT DEVICE     OUTPUT DEVICE     DISPLAY DEVICE
INTERFACE

── 400

┌─ 300

┌─ 101b        ┌─ 102
CONTROL UNIT   MAIN STORAGE
               DEVICE

                                ┌─ 109

┌─ 105         ┌─ 103
COMMUNICATION  AUXILIARY
INTERFACE      STORAGE DEVICE

# FIG. 6

MIXTURE PHYSICAL PROPERTY IDENTIFICATION APPARATUS ⌐100

COMMUNICATION FUNCTION UNIT ⌐120

INPUT FUNCTION UNIT ⌐130

OUTPUT FUNCTION UNIT ⌐140

DISPLAY FUNCTION UNIT ⌐150

STORAGE FUNCTION UNIT ⌐160

CONTROL FUNCTION UNIT ⌐170

PHYSICAL PROPERTY VALUE DATA OBTAINING UNIT ⌐171

PREDICTION TERM CREATION UNIT ⌐172

PHYSICAL PROPERTY IDENTIFICATION UNIT ⌐173

# FIG. 7A

START

DETERMINE PHYSICAL PROPERTY TO BE IDENTIFIED IN MIXTURE ── S201

SELECT PLURAL CANDIDATE SUBSTANCES TO BE MIXED IN MIXTURE ── S202

PREPARE PLURAL MIXTURES EACH CONTAINING TWO OR MORE OF CANDIDATE SUBSTANCES AND OBTAIN DATASETS INDICATING PHYSICAL PROPERTY OF RESPECTIVE MIXTURES (EXPERIMENT, PHYSICAL PROPERTY SIMULATION, OR THE LIKE) ── S203

CONSTRUCT FIRST PREDICTION MODEL USING DATASETS ON PHYSICAL PROPERTY (MULTIVARIATE ANALYSIS USING MULTIPLE REGRESSION EQUATION) ── S204

CALCULATE RMSE/MAE BASED ON PREDICTION VALUES CALCULATED BY USING FIRST PREDICTION MODEL ── S205

(A) ◄── NO ── DOES RMSE/MAE SATISFY 1.253 ± 0.03? ── S206

│YES

CALCULATE REGRESSION COEFFICIENTS OF RESPECTIVE CANDIDATE SUBSTANCES ACCORDING TO FIRST PREDICTION MODEL AND CREATE PREDICTION TERM ── S207

(B)

DEFINE OBJECTIVE FUNCTION EXPRESSION INCLUDING CREATED PREDICTION TERM ── S215

CONVERT OBJECTIVE FUNCTION EXPRESSION TO ISING MODEL OF EXPRESSION (1) AFTER CHANGING WEIGHTING COEFFICIENTS AS REQUESTED ── S216

MINIMIZE EXPRESSION (1) BY USING ANNEALING MACHINE ── S217

OUTPUT KINDS OF CANDIDATE SUBSTANCES INCLUDED IN MIXTURE, PERCENTAGES OF CANDIDATE SUBSTANCES MIXED, AND PHYSICAL PROPERTY OF MIXTURE ── S218

END

...

# FIG. 7B

(A)

| PREPARE PLURAL COMPOSITION-BY-COMPOSITION PREDICTION MODELS BASED ON DATASETS INDICATING PHYSICAL PROPERTY | ~ S208 |

| CREATE INTEGRATION MODEL (GAUSSIAN MIXTURE MODEL) BY INTEGRATING PLURAL PREDICTION MODELS PREPARED TOGETHER | ~ S209 |

| GENERATE PREDETERMINED NUMBER OF VIRTUAL DATASETS BASED ON INTEGRATION MODEL | ~ S210 |

| CONSTRUCT SECOND PREDICTION MODEL BY USING VIRTUAL DATASETS (MULTIVARIATE ANALYSIS USING MULTIPLE REGRESSION EQUATION) | ~ S211 |

| CALCULATE RMSE/MAE BASED ON PREDICTION VALUES CALCULATED BY USING SECOND PREDICTION MODEL | ~ S212 |

S213

NO ← DOES RMSE/MAE SATISFY $1.253 \pm 0.03$?

↓ YES

| CALCULATE REGRESSION COEFFICIENTS OF RESPECTIVE CANDIDATE SUBSTANCES ACCORDING TO SECOND PREDICTION MODEL AND CREATE PREDICTION TERM | ~ S214 |

(B)

# FIG. 8

# FIG. 9

```
┌─────────────────────────────────┐
│  SELECT ONE STATE TRANSITION AS │ ⌇ S0001
│           CANDIDATE             │
└─────────────────────────────────┘
                 │
                 ▼
┌─────────────────────────────────┐
│ DETERMINE WHETHER STATE TRANSITION │
│ IS ACCEPTABLE BY COMPARING ENERGY  │
│  CHANGE VALUE ALONG WITH STATE     │ ⌇ S0002
│    TRANSITION AND PRODUCT OF       │
│  TEMPERATURE VALUE AND RANDOM      │
│          NUMBER VALUE              │
└─────────────────────────────────┘
                 │
                 ▼
┌─────────────────────────────────┐
│   ACCEPT STATE TRANSITION IF IT IS │
│ ACCEPTABLE OR REJECT IT IF IT IS NOT │ ⌇ S0003
│           ACCEPTABLE               │
└─────────────────────────────────┘
```

# FIG. 10

# FIG. 11

# FIG. 12

# FIG. 13

## EUROPEAN SEARCH REPORT

**Application Number**

EP 21 19 6277

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOSSEINZADEH M. ET AL: "Toward a predictive model for estimating viscosity of ternary mixtures containing ionic liquids", JOURNAL OF MOLECULAR LIQUIDS, vol. 200, no. Part B, 27 October 2014 (2014-10-27), pages 340-348, XP055907014, NL ISSN: 0167-7322, DOI: 10.1016/j.molliq.2014.10.033 Retrieved from the Internet: URL:https://www.sciencedirect.com/science/article/pii/S0167732214005054/pdfft?md5=12a71caaad99ef81de5ca59fd0829f4c&pid=1-s2.0-S0167732214005054-main.pdf> * the whole document * * in particular: * * abstract * * paragraph [002.] – paragraph [3.2.] * * figures 1,3,4 * | 1-10 | INV. G16C20/30 G16C60/00 ADD. G16C20/70 |
| X | KLEIN J.A. ET AL: "Computer aided mixture design with specified property constraints", COMPUTERS & CHEMICAL ENGINEERING, vol. 16, no. Suppl 1, 1 May 1992 (1992-05-01), pages S229-S236, XP055911079, GB ISSN: 0098-1354, DOI: 10.1016/S0098-1354(09)80027-6 * the whole document * * in particular: * * abstract * * page S230 – page S234 * | 1-10 | **TECHNICAL FIELDS SEARCHED (IPC)** G16C |

−/−−

The present search report has been drawn up for all claims

| Place of search | Date of completion of the search | Examiner |
|---|---|---|
| The Hague | 9 April 2022 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

EUROPEAN SEARCH REPORT

**Application Number**

EP 21 19 6277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIU Y. ET AL: "Machine learning for predicting thermodynamic properties of pure fluids and their mixtures", ENERGY, vol. 188, 9 September 2019 (2019-09-09), page 116091, XP055907025, AMSTERDAM, NL ISSN: 0360-5442, DOI: 10.1016/j.energy.2019.116091 Retrieved from the Internet: URL:http://www.heatenergist.org/upload/publication/S1917-144%20Energy-Machine%20learning%20for%20predicting%20thermodynamic%20properties%20of%20pure%20fluids%20and%20their%20mixtures.pdf> * the whole document * * in particular: * * abstract * * paragraph [2.1.] - paragraph [2.3.] * * figure 1 * ----- | 1-10 | |
| X | PERDOMO F. A. ET AL: "Predicting the physical-chemical properties of biodiesel fuels assessing the molecular structure with the SAFT-[gamma]group contribution approach", ENERGY, ELSEVIER, AMSTERDAM, NL, vol. 72, 14 June 2014 (2014-06-14), pages 274-290, XP028879474, ISSN: 0360-5442, DOI: 10.1016/J.ENERGY.2014.05.035 * the whole document * * in particular: * * abstract * * paragraph [4.2.] - paragraph [4.2.3.] * ----- -/-- | 1-10 | TECHNICAL FIELDS SEARCHED (IPC) |

The present search report has been drawn up for all claims

| Place of search | Date of completion of the search | Examiner |
|---|---|---|
| The Hague | 9 April 2022 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 2 of 3**

Europäisches
Patentamt

European
Patent Office

Office européen
des brevets

## EUROPEAN SEARCH REPORT

**Application Number**

**EP 21 19 6277**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUPTA S. ET AL: "Industrial Needs in Physical Properties", INDUSTRIAL & ENGINEERING CHEMISTRY RESEARCH, vol. 42, no. 25, 1 December 2003 (2003-12-01), pages 6359-6374, XP055901729, ISSN: 0888-5885, DOI: 10.1021/ie030170v * the whole document * | 1-10 | |
| A | MARCOULAKI E. C. ET AL: "Novel chemicals for clean and efficient processes using stochastic optimization", COMPUTERS & CHEMICAL ENGINEERING, PERGAMON PRESS, OXFORD, GB, vol. 24, no. 2-7, 15 July 2000 (2000-07-15), pages 705-710, XP026981749, ISSN: 0098-1354 [retrieved on 2000-07-15] * the whole document * | 1-10 | |

TECHNICAL FIELDS
SEARCHED      (IPC)

The present search report has been drawn up for all claims

| Place of search | Date of completion of the search | Examiner |
|---|---|---|
| The Hague | 9 April 2022 | Godzina, Przemyslaw |

page 3 of 3

**REFERENCES CITED IN THE DESCRIPTION**

**Patent documents cited in the description**

- JP 2020030680 A

- JP 2019195838 A